# EUROPEAN PATENT SPECIFICATION

(11) **EP 1 163 241 B1**
(45) Date of publication and mention of the grant of the patent: **15.06.2005**
(21) Application number: 00913159.0
(22) Date of filing: 13.03.2000
(51) Int. Cl.: C07D 471/04, A61K 31/437, A61P 25/20

(54) **ZOLPIDEM SALTS**
ZOLPIDEM SALZE
SELS DE ZOLPIDEM

(30) Priority: 25.03.1999 US 126494 P; 22.10.1999 EP 99203478; 26.11.1999 US 449974
(43) Date of publication of application: 19.12.2001
(73) Proprietor: Synthon B.V., 6545 CM Nijmegen (NL)
(72) Inventor: ETTEMA, Gerrit, Jan, Bouke, NL-7591 ER Denekamp (NL); LEMMENS, Jacobus, Maria, NL-6585 KM Mook (NL); PETERS, Theodorus, Hendricus, Antonius, NL-6814 JB Arnhem (NL); PICHA, Frantisek, 615 00 Brno (CZ)
(74) Representative: Duxbury, Stephen
(86) International application number: PCT/NL2000/000171
(87) International publication number: WO 2000/058310

(56) References cited:
- EP-A- 0 251 859
- EP-B- 0 050 563
- DATABASE CAPLUS [Online] Chemical Abstracts Service; Abstract 105:35459, XP002144588 & JOURNAL OF PHARMACOLOGY AND EXPERIMENTAL THERAPEUTICS., vol. 237, no. 2, 1986, pages 659-65, BALTIMORE, MD., US ISSN: 0022-3565 & CHEMICAL ABSTRACTS, vol. 105, no. 5, 1986 Columbus, Ohio, US; abstract no. 35459v, B. SCATTON: "Zolpidem, a novel nonbenzodiazepine hypnotic. II. Effects on cerebellar cyclic GMP levels and cerebellar monoamines" page 54;

## Description

The present invention relates to novel salts of zolpidem and to pharmaceutical compositions and methods of treatment containing the same.

Zolpidem, an imidazopyridine having the IUPAC chemical nomenclature N,N,6-trimethyl-2-(4 methylphenyl)-imidazo[1,2-a]pyridine-3-acetamide, is a known hypnotic agent having the following formula.

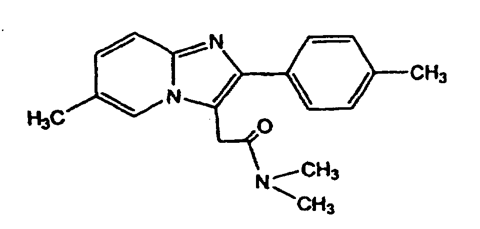

It has been marketed in solid dosage forms for peroral application (tablets) under the trademarks AMBIEN® and STILNOX®. As the active substance in these pharmaceutical dosage forms, zolpidem is present in the form of a salt with natural L(+)tartaric acid ((2R,3R)-2,3-dihydroxybutanedicarboxylic acid) wherein the molar ratio of zolpidem and tartaric acid in the salt is 2:1. This salt is conventionally called zolpidem hemitartrate but more a correct denomination thereof, which will be used hereinafter, is zolpidem tartrate.

EP 50563 discloses imidazo[1,2-a]pyridines to which zolpidem free base belongs, and there pharmaceutically acceptable acids of addition.

CA 35459v, vol.105, no. 5,1986 discloses the use of zolpidem hemitartrate as a non-benzodiazepine short-acting hypnotic.

Zolpidem tartrate, so far the only known salt of zolpidem, is disclosed in Journal of Pharmacology and Experimental Therapeutics 237(2), 659-65 (1986) [CA 105:35459v] and more particularly, in EP 251859 (U.S. Patent 4,794,185) together with a synthesis scheme therefor. An example therein shows the production of zolpidem and the formation of the tartrate thereof. Specifically, 25 g of zolpidem free base is dissolved in 180 ml of methanol and combined with 60 ml of a methanol solution containing 6.1 g of tartaric acid (a 2:1 molar ratio) and then allowing the mixed solution to crystallize. The crystalline product is reported to have a melting point of 197°C. The specific details of how the crystallization is performed are not disclosed.

The European Pharmacopoeia, Monograph No. 1999:1280, states that zolpidem tartrate is characterized as a white or almost white crystalline powder, hygroscopic, slightly soluble in water, sparingly soluble in methanol, and practically insoluble in methylene chloride. For identification purposes, a Pharmacopoeial reference standard, namely zolpidem tartrate CRS, is a commercially available substance that serves as the reference for any analysis or comparison.

Studies conducted by the inventors have revealed that forming the zolpidem tartrate salt is physically difficult and that the zolpidem tartrate salt is not physically stable in its solid state.

The method of manufacturing zolpidem tartrate described in the documents discussed above comprises crystallization of a mixed solution of zolpidem free base and tartaric acid (2:1 molar ratio) in methanol.

The inventors have repeated this method with the aim to test the ruggedness of the production method in modeling situations encountered on an industrial scale (changes in temperature regimen, concentration of components, quality of solvent used, etc.) and found out that the crystallization process is highly irreproducible. Proper formation of crystalline zolpidem tartrate in desired yield and quality is highly dependent on strict control and maintaining the optimum crystallization conditions, quality of starting materials (namely content of water in methanol) and molar ratio of zolpidem and tartaric acid. It often happened that, from batch to batch and under otherwise the same conditions, crystals were not formed at the preselected temperature in a reasonable time and it was necessary to continue with cooling the mixture to lower temperatures to obtain a solid product. At temperatures lower than ambient, impurities present in the starting materials can also coprecipitate and thus decrease the quality of the obtained product. Also, and sometimes unpredictably, another salt is obtained instead of the desired tartrate. Thus, it is highly unpredictable to estimate whether and when the desired product will be obtained from the solution and what the yield and quality of the product from a production batch will be.

Moreover the inventors have found that the known zolpidem tartrate has low physical stability. That is, the application of energy such as mechanical grinding, heat, etc., to the known zolpidem tartrate can readily cause the crystal structure to change forms and in particular can cause the release of zolpidem free base.

This physical instability can lead to unintended changes in the zolpidem salt form during manufacture or storage. For example, during storage the zolpidem tartrate could begin to decompose into a physical mixture of zolpidem hydrogen tartrate (a salt having a 1:1 molar ratio of zolpidem to tartaric acid) and zolpidem free base. The zolpidem free base is not as water soluble as the zolpidem tartrate or zolpidem hydrogen tartrate and thus may not be taken up by the body in vivo thereby reducing the bioavailability.

Accordingly, it is an object of the present invention to provide a zolpidem salt form that exhibits greater physical stability than the known zolpidem tartrate and is preparable in reproducible manner, especially on an industrial scale.

According to a first aspect of the present invention there is provided a zolpidem salt form, as claimed in claim 1.

The inventors have shown that the novel zolpidem salt forms according to the present invention do not exhibit the stability problems of the known zolpidem tartrate.

A zolpidem salt according to the present invention most preferably exhibits a single melting endotherm when heated.

In contrast, the known zolpidem tartrate form will exhibit two melting endotherms: one for the zolpidem hydrogentartrate and one for the zolpidem free base which is released during the heating process. This indicates a change of crystalline structure under elevated temperature, which can lead to loss in the original pharmaceutical characteristics.

The solid-phase zolpidem salts of the present invention have sufficient physical stability in solid state that upon heating from about 20°C to about 250°C at a rate of about 5°C/minute, they do not exhibit a melting endotherm that corresponds to zolpidem free base.

The inventors theorize that by forming zolpidem salts containing strong ionic bonds between zolpidem and acid anion moieties, a zolpidem salt form is obtained that exhibits superior physical stability in comparison to the known zolpidem tartrate salt form.

One way to obtain such a salt is by having a molar ratio of protonated zolpidem moiety to anion in the range of about 0.9-1.35:1, more preferably about 1:1. Such salts represent another aspect of the present invention. The salts according to the present invention can be made from pharmaceutically acceptable acid addition salts such as hydrochloride, hydrobromide, maleate, fumarate, tartrate, sulfate and sulfonates.

The zolpidem salts according to the present invention may be crystalline or amorphous and they may contain bound water or solvent or be substantially free of the same. Preferred salts include various forms of zolpidem hydrogentartrate, zolpidem hydrochloride, and zolpidem methane sulfonate.

The invention also relates to the use of the physically stable zolpidem salts of the present invention as intermediates in the preparation of another zolpidem salt including the known zolpidem tartrate.

The zolpidem salt forms of the present invention are useful in pharmaceutical compositions and for the treatment of mammals such as for inducing sleep or drowsiness. The pharmaceutical compositions can be solid dosage forms or liquid dosage forms containing the zolpidem salt dissolved therein.

Upon heating from about 20°C to about 250°C at a rate of about 5°C/minute, none of the zolpidem salt forms of the present invention exhibits a melting endotherm that corresponds to zolpidem free base. In practice this means that the zolpidem salt form does not disproportionate due to the application of heat to liberate a zolpidem free base prior to melting. Instead, the zolpidem salts of the present invention are thermally stable; i.e. no appreciable liberation of a zolpidem free base. The above heating test is usually carried out in a differential scanning calorimeter (DSC) under a nitrogen atmosphere at atmospheric pressure.

Alternatively, the heating test may be performed using differential thermal analysis (DTA).

Normally the zolpidem salt forms of the present invention exhibit only one melting endotherm. However, it is possible that a salt form will have two or more melting endotherms and still not release a free base of zolpidem. For instance, the salt form may melt and recrystallize as a new crystal form and then melt again, but without releasing the zolpidem free base. Such a composition would have two melting endotherms but neither of them would correspond to a melting endotherm for zolpidem free base. Similarly, a salt could decompose prior to melting into decomposition products; if zolpidem free base is not formed thereby, such salt is also within the scope of the invention.

In contrast, the conventional zolpidem tartrate exhibits two melting endotherms because the composition is not thermally stable and during the heating process is primarily converted into a physical mixture of zolpidem hydrogen tartrate and zolpidem free base. The first melting endotherm corresponds to zolpidem free base while the second corresponds to zolpidem hydrogentartrate.

The zolpidem salts of the present invention generally display good physical stability to a variety of energy inputs. For example, salts according to the present invention are generally stable against a variety of heat treatments including long term exposure to constant low level heating.

The same is not true of the conventional zolpidem tartrate. Similarly, the zolpidem salts of the present invention are generally physically stable to ultrasound exposure and mechanical stresses such as grinding and compression. That is, after being subjected to these treatments, a zolpidem free base is not appreciable released. Zolpidem free base is considered to be "appreciably released" or appreciably present in the composition when the zolpidem free base provides a visually discernable peak on an IR, DSC, or X-ray powder diffraction graph. Generally a peak is not visually discernable when the amount of zolpidem free base is less than 2% and preferably less than 1% of the zolpidem moieties present in the composition.

In contrast to the present invention, the application of energy to the known zolpidem tartrate such as by heating results in a composition that is different from zolpidem tartrate but indistinguishable from an artificially made admixture of zolpidem hydrogentartrate and zolpidem free base in terms of IR and X-ray powder diffraction. Surprisingly, the same solid-state decomposition has been observed when zolpidem tartrate was subjected to milling, long-term drying or by an action of ultrasound even at temperatures close to ambient. Examination of such treated zolpidem tartrate by IR spectrum or X-ray powder diffraction analysis, shows that the product turned, at least partly, into the physical mixture of zolpidem free base and zolpidem hydrogentartrate. The same behavior could be expected during the tabletting process wherein local overheating is commonly encountered during the homogenization and/or compression (pressurization) of the tablet composition. Indeed, the tabletting process is one of the most common reasons for decomposition of sensitive compounds in the final dosage form.

The solid-phase zolpidem salt forms of the present invention may be crystalline or amorphous. Generally, the ratio of zolpidem to anion is significantly less than 2:1 in the salt form, such as within the range of 0.9-1.35:1, more typically around 1:1. The anion is a pharmaceutically acceptable acid addition salt. The salts include hydrochloride, hydrobromide, maleate, fumarate, tartrate, sulfate and sulfonates such as mesylate and tosylate. The salt can be formed by combining a zolpidem source and an anion source in a solvent to form a solution that contains at least one of the zolpidem and the anion source dissolved therein; and recovering a solid zolpidem salt from the solution.

Surprisingly the inventors have found that the stability of the zolpidem salts, apart from the tartrate, is not influenced by the choice of solvent, see later.

The solvent is typically an alcohol, ketone, hydrocarbon, chlorinated hydrocarbon or water. The zolpidem source is usually a zolpidem free base although other sources such as salts or precursors thereof can also be used. The anion source is generally a pharmaceutically acceptable acid, preferably an acid selected from the group consisting of HCl, HBr, maleic acid, fumaric acid, sulfuric acid, tartaric acid, and alkyl- or aryl-sulfonic acid. However, when the zolpidem source is zolpidem free base and the anion source is tartaric acid, it is preferable that methanol is not the solvent. More generally, when the zolpidem source is zolpidem free base, it is usually desirable that a non-methanolic solvent be used. The recovery step can be carried out by crystallization, with or without ultrasound, freeze drying, spray drying or other known methods.

A preferred salt of the present invention is zolpidem hydrochloride. This salt may be formed in several crystalline forms in dependence on the method of salt formation. However, all the modifications described below fall under the scope of the invention, i.e. they do not decompose into zolpidem free base and hydrochloric acid under action of energy.

Crystalline zolpidem hydrochloride monohydrate may be obtained by crystallization of an equimolar mixture of zolpidem and concentrated hydrochloric acid from their solution in acetone. The melting point of the product when determined on Buchi melting point apparatus is around 277°C.

The DCS curve shows that this salt form releases water at approximately 120°C to form an anhydrous zolpidem hydrochloride, which then has a single melting endotherm at 282°C. The content of water corresponds to one molar equivalent and, as apparent from the elevated release temperature, water is bound in the crystalline lattice quite firmly.

Anhydrous crystalline zolpidem hydrochloride may be obtained by crystallization from n-butanol. This salt form exhibits a single melting endotherm on DSC at 282°C and the melting point, in accordance with the above comment to the monohydrate, is also around 277°C.

When crystallizing zolpidem hydrochloride from an ethanolic solution, a compound is obtained which has yet another peak on DSC at approximately 86°C. Chemical analysis reveals that the produced salt form is a solvate with the ethanol content corresponding to a half molar equivalent. Thus, the product can be characterized as zolpidem hydrochloride hemiethanolate.

The melting endotherm on DSC is, similarly as above, approximately 282°C which indicates that, after liberating ethanol, this salt form also melts without decomposition into zolpidem free base.

Another form of zolpidem hydrochloride may be prepared by passing hydrogen gas through a solution of zolpidem free base in a suitable solvent. In this case, even less polar solvents, such as dichloromethane, may be suitably employed.

Amorphous zolpidem hydrochloride may be prepared by dissolution of a crystalline salt form such as any of the above salt forms, in water and freeze drying of the resulting solution.

Another advantageous salt form of the present invention are zolpidem sulfonates, especially methane sulfonate salt or mesylate. In general these salt forms may be prepared by dissolving zolpidem and methane sulfonic acid (or other alkyl- or aryl-sulfonic acid) in methanol, evaporating the solvent to dryness and crystallization of the resulting mixture from acetone.

Zolpidem methane sulfonate exhibits on DSC curve a single melting endotherm at 206°C. Similarly, zolpidem p-toluene sulfonate may be prepared which exhibits a single melting endotherm at 219°C.

In the production of the zolpidem salts of the present invention, starting zolpidem may be obtained by synthetic methods in the prior art e.g. in EP 50563 and EP 251859 and also by the methods described in commonly owned U.S. patent application 60/126,494.

According to the present invention, there is also provided a zolpidem tartrate salt form which does not suffer from the problems of the known zolpidem tartrate.

The inventors have found that presence of a layer of unprotonated zolpidem should be avoided when manufacturing the zolpidem salts according to the present invention and that, when manufacturing zolpidem tartrate according to the present invention, methanol should also be avoided as a solvent.

Regarding the methanol solvent, the inventors have found that methanol plays a very important role in forming the conventional zolpidem tartrate. Surprisingly, in trying to prepare zolpidem tartrate by crystallization, using a 2:1 molar ratio of zolpidem and tartaric acid and employing non-methanolic solvents such as ethanol, isopropanol and acetone, no zolpidem tartrate was formed. Instead, only zolpidem hydrogentartrate is obtainable. This salt has not been described theretofore and is discusses in more detail hereinafter. Analysis of freshly prepared zolpidem tartrate indicates that it is in fact a solvate with methanol. Two molecules of methanol per one molecule of zolpidem tartrate are present herein.

Methanol, it seems, is an important partner to zolpidem and tartaric acid molecules in the process of crystallization and forming the crystalline lattice of the known zolpidem tartrate.

The methanol can be removed from the lattice, at least in part, by conventional drying or by prolonged storage at ambient temperatures. Once methanol leaves, the lattice becomes quite sensitive and is readily disturbed by the application of energy resulting in the breakdown of the lattice and the release of zolpidem free base.

In non-methanolic solvents, the inventors theorize that a molecule of solvent is probably too large to be included into such an oriented crystalline lattice and thus the crystals of zolpidem tartrate are not formed.

Also, it appears that the water and/or impurities present in methanol play a role in forming the crystalline lattice. This may also explain the unexpected failures in producing zolpidem tartrate as, instead, zolpidem hydrogentartrate has been sometimes obtainable from methanol, even when using a proper 2:1 molar ratio.

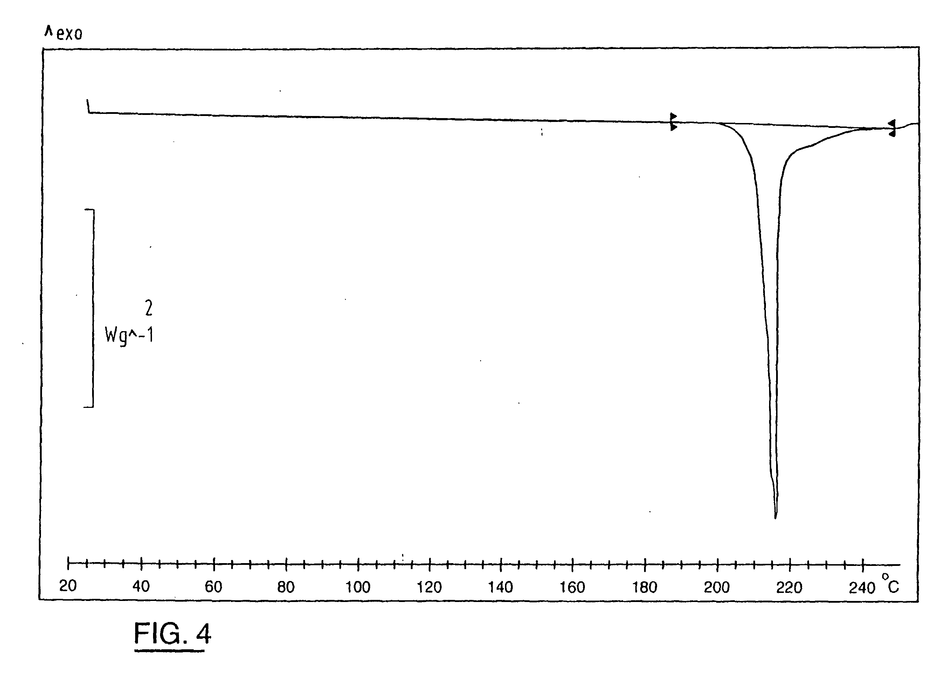

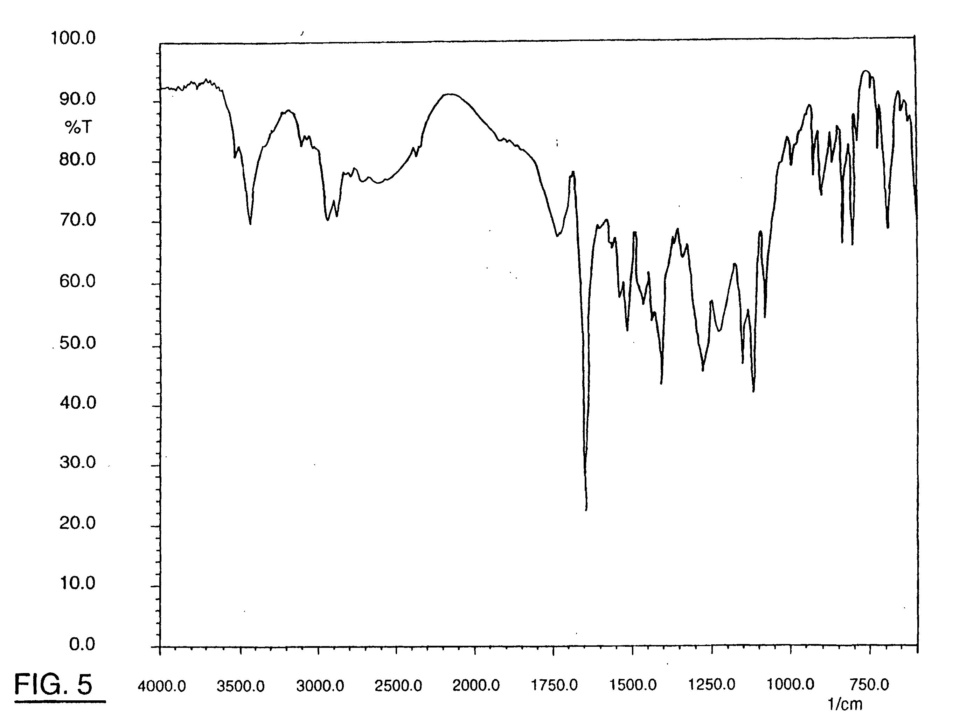

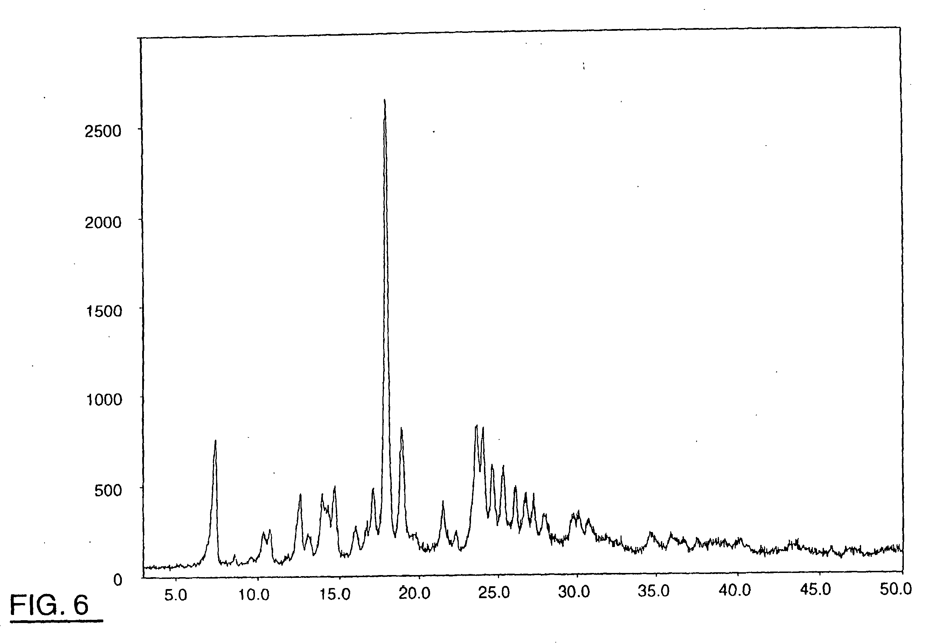

Salts made from tartaric acid according to the present invention can be formed that exhibit physical stability such that a zolpidem free base is not released. Typically these salts are crystalline although amorphous forms can also be made. A salt having a zolpidem to tartaric acid molar ratio of 1:1 is zolpidem hydrogentartrate. In a preferred aspect, the zolpidem hydrogentartrate exists in a crystalline form and more preferably having the following characteristics:
m.p.: 203-204°C
DSC (5°C/min): single melting endotherm at around 209-211°C as shown in fig. 4.
IR (KBr): as shown in fig. 5.
X-ray powder diffraction: as shown in fig. 6.

A product that substantially consists of this zolpidem hydrogentartrate crystalline salt can be prepared in reproducible manner and in industrial scale as will be shown herein below. The words "substantially consists" as used above means that the content of the zolpidem hydrogentartrate in the isolated product is more than 95% pure, preferably more than 98% pure. This purity is advantageous in as much as the intended use of the product is as a pharmaceutically active agent.

It is preferred that the above product is produced substantially free from the bound solvent; i.e. less than 5% preferably less than 2% and more preferably less than 1% solvent. However, the product may be hydrated to a limited extent as it may absorb water after prolonged standing under environment of higher humidity. Preferably, the product contains less than 3% of water. Water and/or bound solvents arising from production may be removed by conventional drying and the resulted product is still stable towards changes in crystalline structure after heating.

The zolpidem hydrogentartrate can be formed when mixing zolpidem free base and tartaric acid in the appropriate solvent such as ethanol, acetone or isopropanol, preferably in amounts close to theoretical (1:1 molar ratio) but it is also formed when mixing zolpidem free base with an excess of tartaric acid (e.g. up to 1:3 molar ratio) or deficiency of tartaric acid (e.g. up to 3:1 molar ratio). Generally, any of the solvents in which both zolpidem and tartaric acid are at least partly soluble is a suitable solvent, and in reality so long as at least one of the zolpidem or tartaric acid is partly soluble in the solvent, salt formation can proceed, albeit much more slowly.

This also illustrates advantages in preparation of crystalline zolpidem hydrogentartrate of the invention as it may be formed under conditions that do not require careful control of crystallization conditions, quality of solvents, quality of the starting materials and their mutual ratio. The compound may thus be produced in a reproducible manner with reliable results in industrial scale.

Although the zolpidem hydrogentartrate is conventionally formed as a crystal, an amorphous form can also be made. Specifically, by subjecting the above crystalline zolpidem hydrogentartrate to freeze drying from a water solution, a different form of zolpidem hydrogen tartrate is obtained. This form has the same chemical composition as the starting zolpidem hydrogentartrate but it has no defined X-ray powder diffractogram and it is thus an amorphous zolpidem hydrogentartrate. Physical stability in solid state is the same for the amorphous form as in the case of crystalline compound.

Amorphous zolpidem hydrogentartrate is formed as microparticulate solid matter with low bulk density so that it dissolves rapidly and reliably in pharmaceutically acceptable solvents (carriers or diluents) such as water or physiological saline. Accordingly, the amorphous form is useful in formulating liquid dosage forms such as for injections.

In addition to zolpidem hydrogentartrate, other tartrate salts can be formed by controlling the salt formation conditions. For example, forms having a ratio other than 1:1 can be made including salt form where the molar ratio of zolpidem to anion is 4:3 (or 1.33:1).

All the above described inventive zolpidem salt forms are useful as pharmaceuticals in the same therapeutic category as the known zolpidem tartrate and they can be formulated into conventional pharmaceutical dosage forms. In particular, zolpidem salt forms can be used as hypnotics to aid in sleep disorders or in any application where sleep or drowsiness is desired. More recently, zolpidem has been suggested for use in treating Parkinson's disease, parkinsonian symptoms, obsessive-compulsive disorder and certain dementias in U.S. Patent 5,891,891, the entire contents of which are incorporated herein by reference. The zolpidem salts can be administered in dosage forms and routes well known in the art. Generally, the pharmaceutical composition comprises a pharmaceutically acceptable carrier or diluent and an effective amount, e.g. a hypnotically effective amount, of one of the above described novel zolpidem salt forms. The amount of zolpidem tartrate as effective for a given condition or treatment is well known in the art and/or can be readily ascertained by workers skilled in the art. Generally the zolpidem salt form is contained in an amount within the range from about 5 mg to 50 mg per unit dose. The dosage forms include solid as well as liquid and can be oral or parenteral. In one embodiment the composition comprises a pharmaceutically acceptable liquid carrier having an effective hypnotic amount of the zolpidem salt according to the present invention dissolved therein. This composition can be either for oral administration or for injection. The liquid carrier is preferably water or ethanol or a combination thereof. Preferably, the composition is a tablet, a capsule, or a liquid. Preferred salts include zolpidem hydrogentartrate, zolpidem hydrochloride, zolpidem mesylate, zolpidem tosylate, and zolpidem hydrogensulfate, and more particularly amorphous zolpidem hydrogentartrate, crystalline zolpidem hydrogentartrate anhydrate, amorphous zolpidem hydrochloride, crystalline zolpidem hydrochloride monohydrate, crystalline zolpidem hydrochloride anhydrate, crystalline zolpidem hydrochloride hemi-ethanoate, crystalline zolpidem (1:1) sulfate, or zolpidem hydrogensulfate, and crystalline zolpidem mesylate.

A typical tablet formulation may contain zolpidem hydrochloride monohydrate, lactose, microcrystalline cellulose, hypromellose, carboxymethylstarch sodium and magnesium stearate. The tablets may be coated by conventional coating techniques.

Further, zolpidem salts, especially those that have good water solubility can be formulated into parenteral pharmaceutical dosage forms such as injectable compositions. Such injections should preferably consist of a sterile aqueous solution of the corresponding salt of zolpidem, with or without common excipients such as sodium chloride to avoid hemolysis and a physiologically acceptable buffer. In the following table, the solubility of zolpidem and various salts thereof in water at 20°C are given in mg/ml:

| | |
|---|---|
| Zolpidem | 0.12 |
| Zolpidem tartrate | 18.8 |
| Zolpidem hydrogentartrate | 19.9 |
| Zolpidem tosylate | 22.2 |
| Zolpidem hydrochloride monohydrate | 110.0 |
| Zolpidem hydrogensulfate | 150.4 |
| Zolpidem mesylate | 432.0 |

It is apparent, zolpidem hydrochloride, hydrogensulfate, and mesylate (methane sulfonate) are examples of salts which are easy to be formulated into liquid parenteral forms and are preferred for use in such applications.

The dosage amounts of the active substances in the said pharmaceutical compositions and useful therapeutical regimens should advantageously correspond to the already recommended amounts and regiments of the known zolpidem tartrate but they are not limited thereto. The zolpidem salt form can be administered in an effective amount, by any of the above-described or known means, to an animal, particularly to a mammal in an effective hypnotic amount, i.e. effective to induce unconsciousness or drowsiness and preferably unconsciousness.

Beyond the pharmaceutical application, zolpidem salt forms according to the present invention, especially zolpidem hydrogentartrate, can also be used as intermediate in the formation of other or known salts of zolpidem. In particular, the known zolpidem tartrate can be made by a process that comprises combining zolpidem hydrogentartrate with a zolpidem free base in molar ratio of 1:1 in methanol to form a methanolic solution having a temperature of at least 50°C and then cooling the methanolic solution to precipitate solid zolpidem tartrate. Furthermore, an amorphous form of zolpidem tartrate can be made by mixing of zolpidem hydrogentartrate, either in solid form or in solution, with zolpidem free base in a proper solvent such as water or alcoholic solvent in a molar ratio 1:1 and separating the solid product by freeze drying and/or spray drying. The amorphous zolpidem tartrate possesses advantageous properties to that of the known crystalline form as it is formed in microparticulate form which has low bulk density. Therefore it dissolves more quickly in water, physiological saline, alcohol and similar pharmaceutically acceptable solvents and this fact is advantageous namely for industrial production of liquid pharmaceutical dosage forms. In oral pharmaceutical formulations such as tablets, it dissolves more rapidly than the crystalline compound so it may provide better bioavailability after peroral administration.

More generally, all of the zolpidem salts of the present invention can be used as intermediates in the formation of another zolpidem salt. By forming a zolpidem salt of the present invention, especially a salt that is easily crystallizable, a more pure zolpidem product can be obtained. The salt formed in the crystallization can be converted to another salt directly or by first liberating zolpidem free base followed by salt formation. Such an intermediate crystallization process step represents an efficient method of purification of either a zolpidem free base or a zolpidem salt. Accordingly, any salt of the present invention is also useful in the preparation of another salt of zolpidem.

The invention will be further illustrated by way of the following examples having reference to the accompanying figures wherein:
Fig. 1 shows a DCS curve of standard zolpidem tartrate;
Fig. 2 shows an IR spectrum of standard zolpidem tartrate;
Fig. 3 shows an X-ray powder diffraction pattern of standard zolpidem tartrate;
Fig. 4 shows a DSC curve of zolpidem hydrogentartrate;
Fig. 5 shows an IR spectrum of zolpidem hydrogentartrate;
Fig. 6 shows an X-ray powder diffraction pattern of crystalline zolpidem hydrogentartrate;
Fig. 7 shows an X-ray powder diffraction pattern of a heat treated zolpidem tartrate salt;
Fig. 8 shows a comparison of X-ray powder diffraction patterns for a heat treated zolpidem tartrate (graph A) and an equimolar mixture of zolpidem free base and zolpidem hydrogentartrate (graph B);
Fig. 9 shows a DSC curve for amorphous zolpidem hydrogentartrate;
Fig. 9A shows an NMR spectrum of the amorphous zolpidem hydrogentartrate from example 6;
Fig. 9B shows an infra-red spectrum of the amorphous zolpidem hydrogentartrate from example 6;
Fig. 10 shows a DSC curve for zolpidem hydrochloride monohydrate;
Fig. 11 shows a DSC curve for zolpidem hydrochloride anhydrate;
Fig. 12 shows a DSC curve for zolpidem mesylate from example 10;

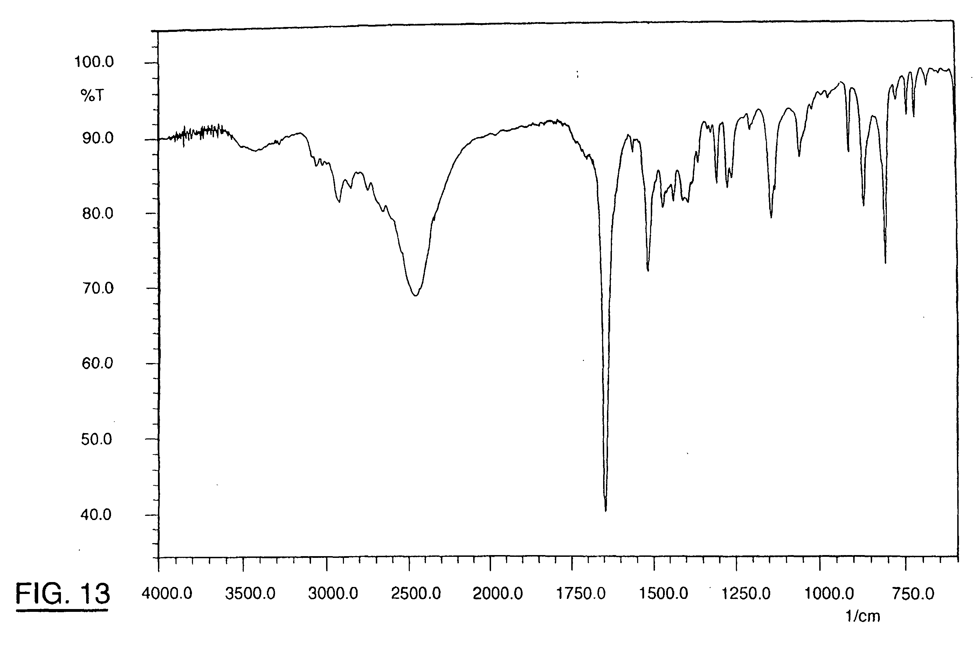

Fig. 13 shows an infra-red spectrum for the zolpidem hydrochloride anhydrate from example 8;

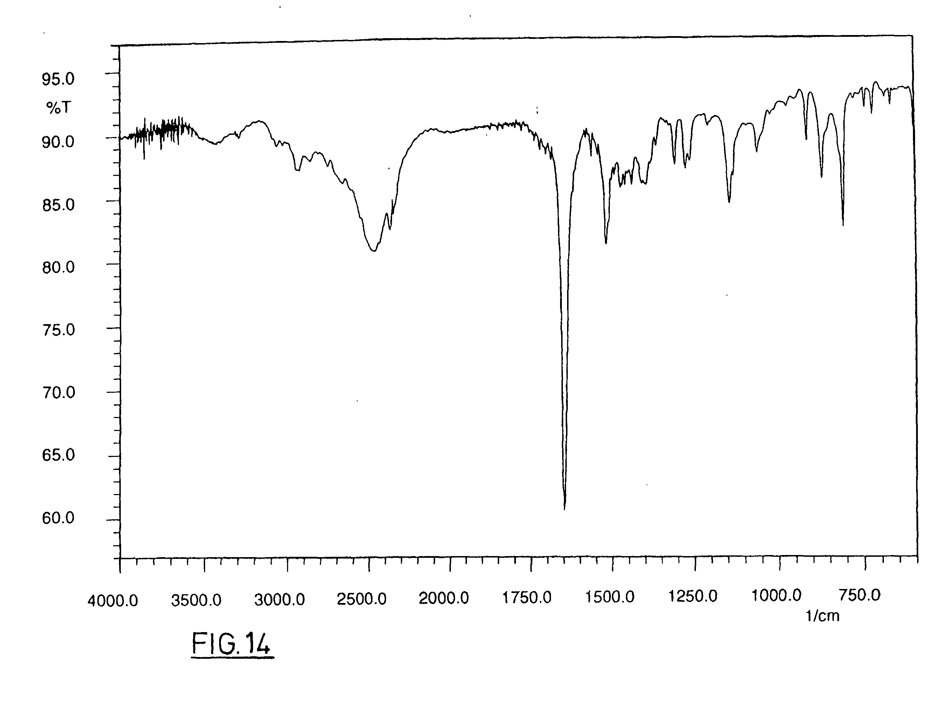

Fig. 14 shows an infra-red spectrum for the zolpidem hydrochloride anhydrate from example 8 measured after exposure to air for 1 hour at 20°C;

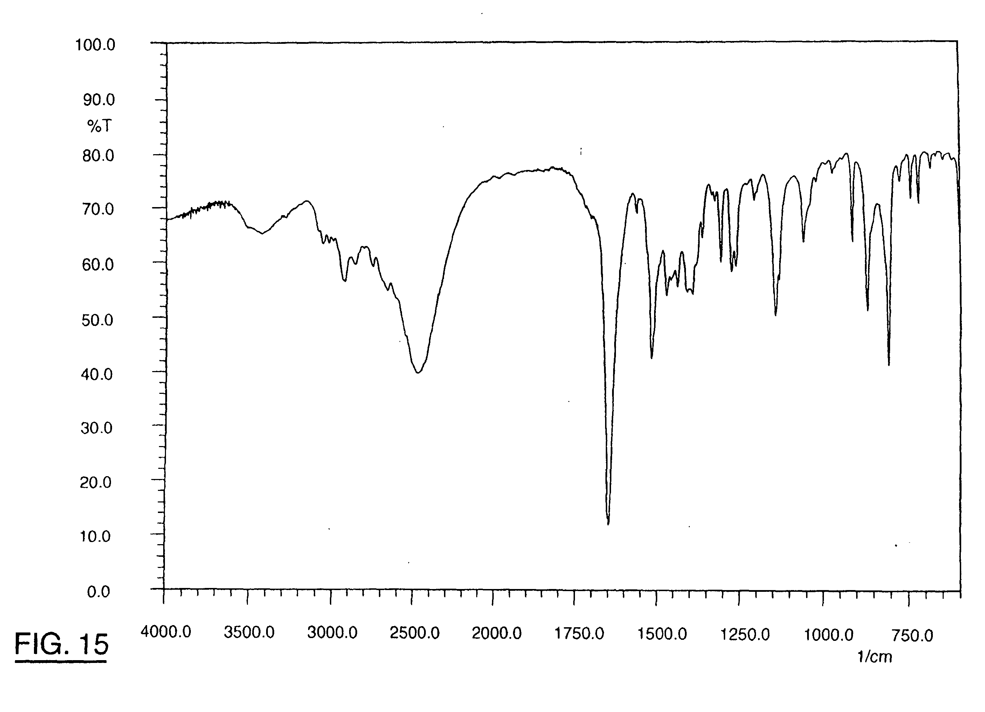

Fig. 15 shows an infra-red spectrum for the zolpidem hydrochloride anhydrate from example 8 measured after exposure to air for 16 hours at 20°C;

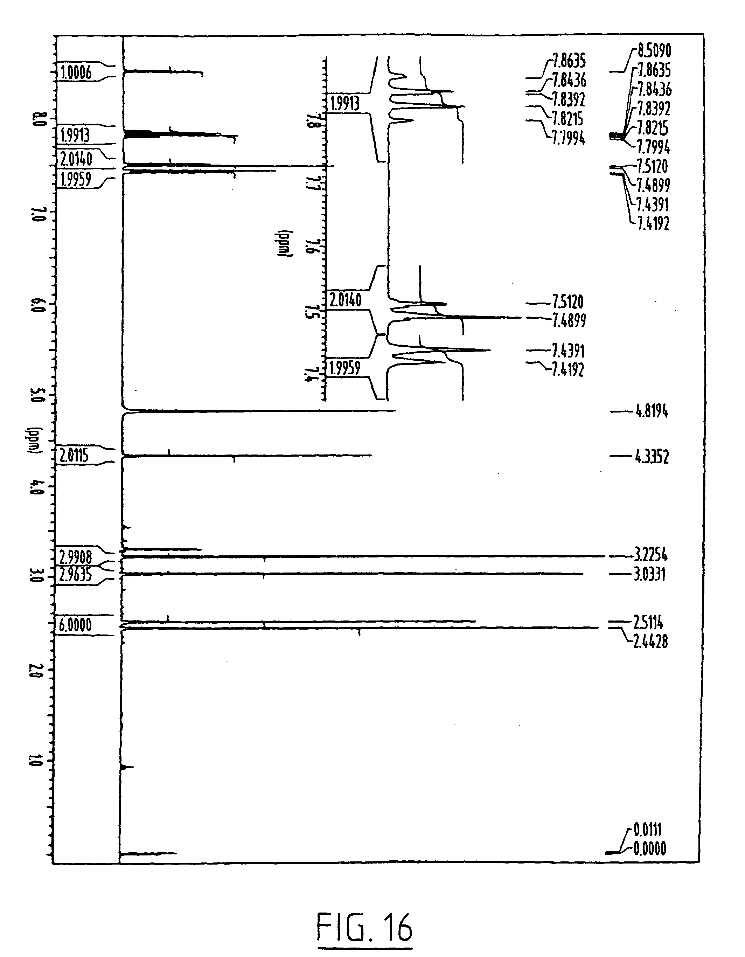

Fig. 16 Shows the NMR spectrum of the zolpidem hydrochloride anhydrate from example 8;

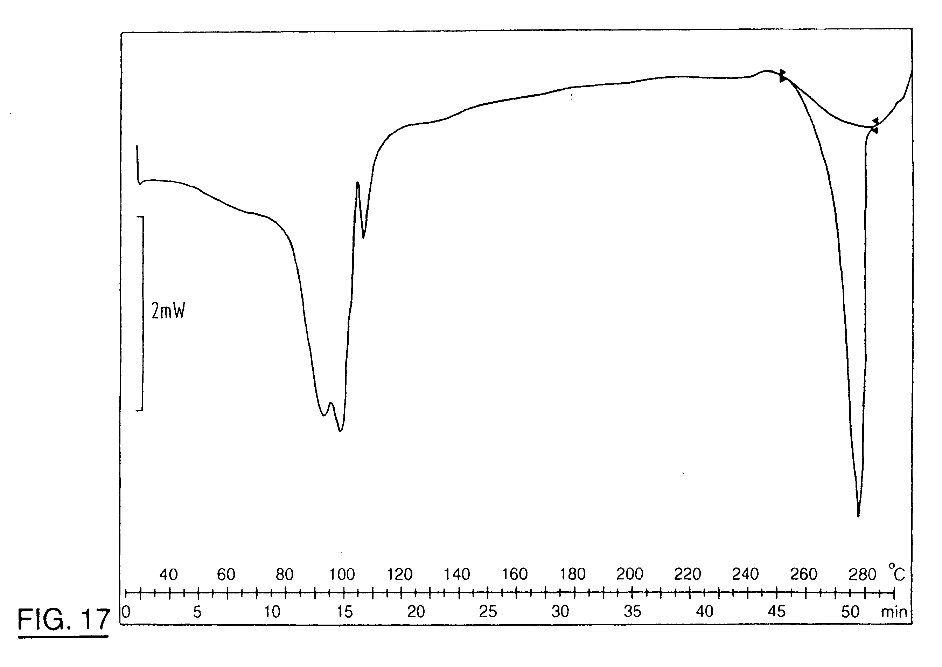

Fig. 17 shows a DSC curve of the zolpidem hydrochloride from example 11;

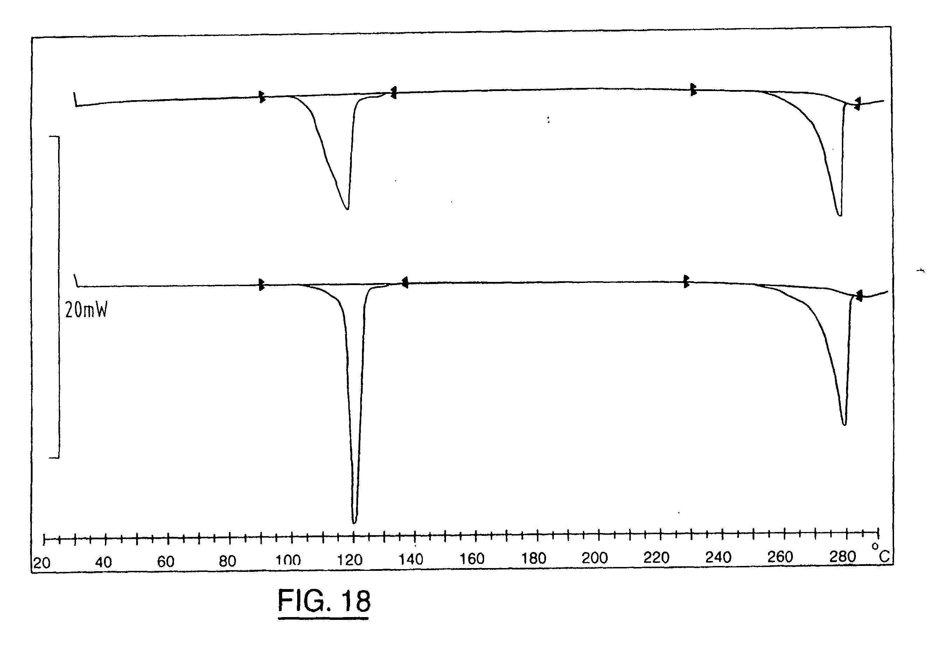

Fig. 18 shows DSC curves of the zolpidem hydrochloride monohydrate from example 12;

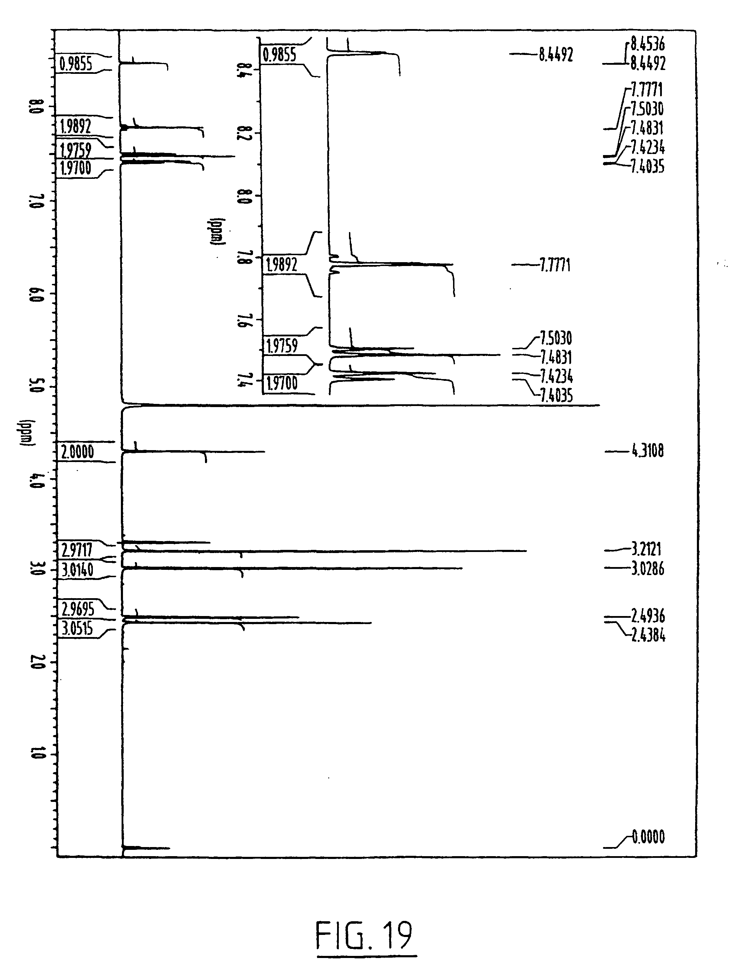

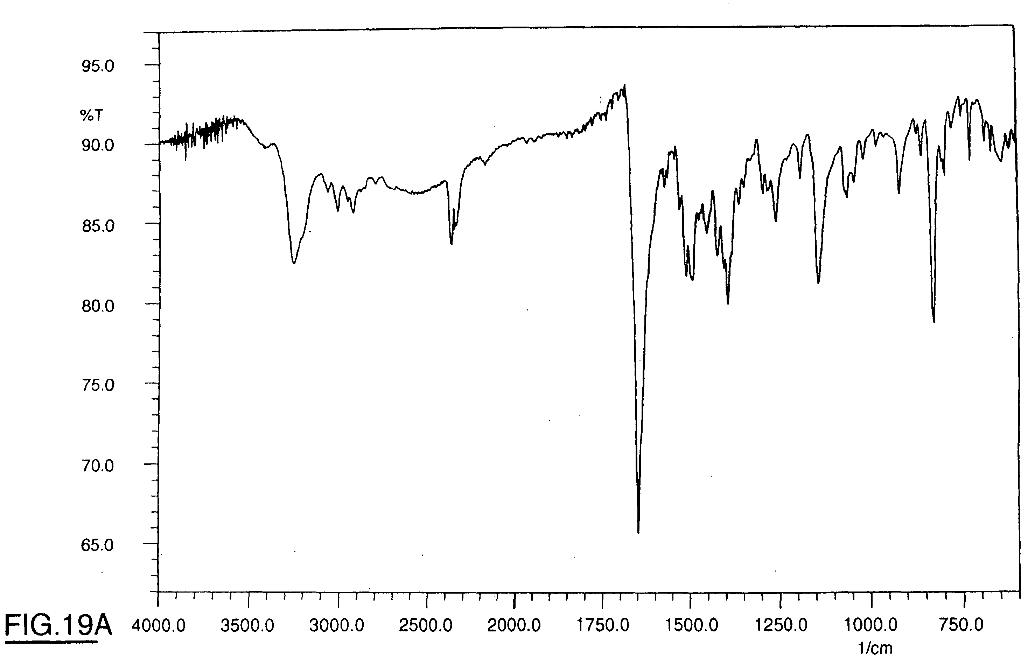

Fig. 19 shows an NMR spectrum of zolpidem hydrochloride monohydrate from example 7;
Fig. 19A shows an IR spectrum of the zolpidem hydrochloride monohydrate from example 7;

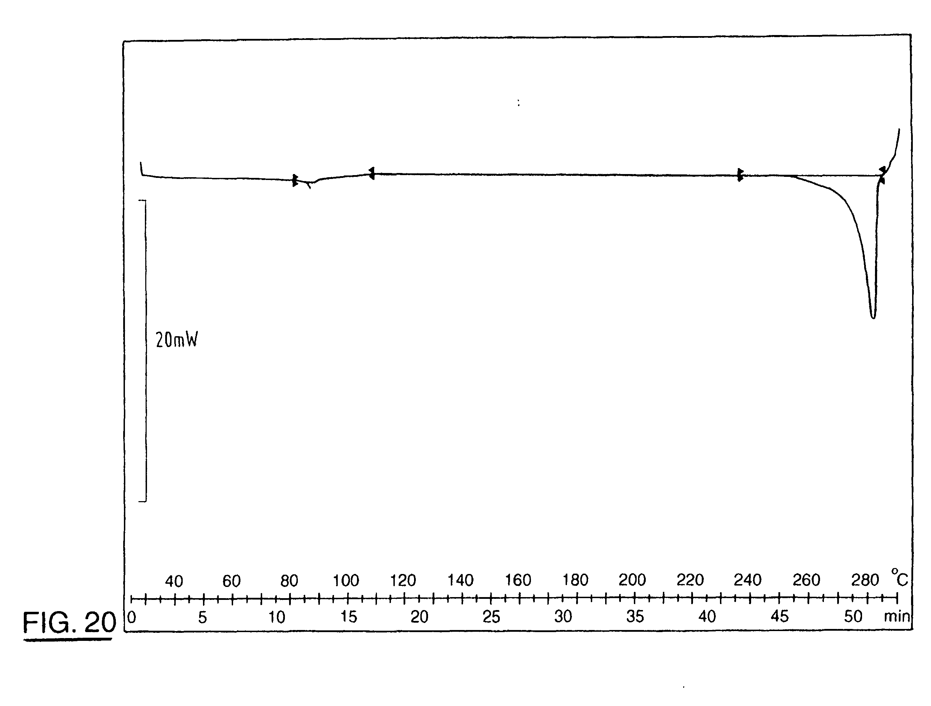

Fig. 20 shows a DSC curve of the zolpidem hydrochloride hemiethanolate from example 9;

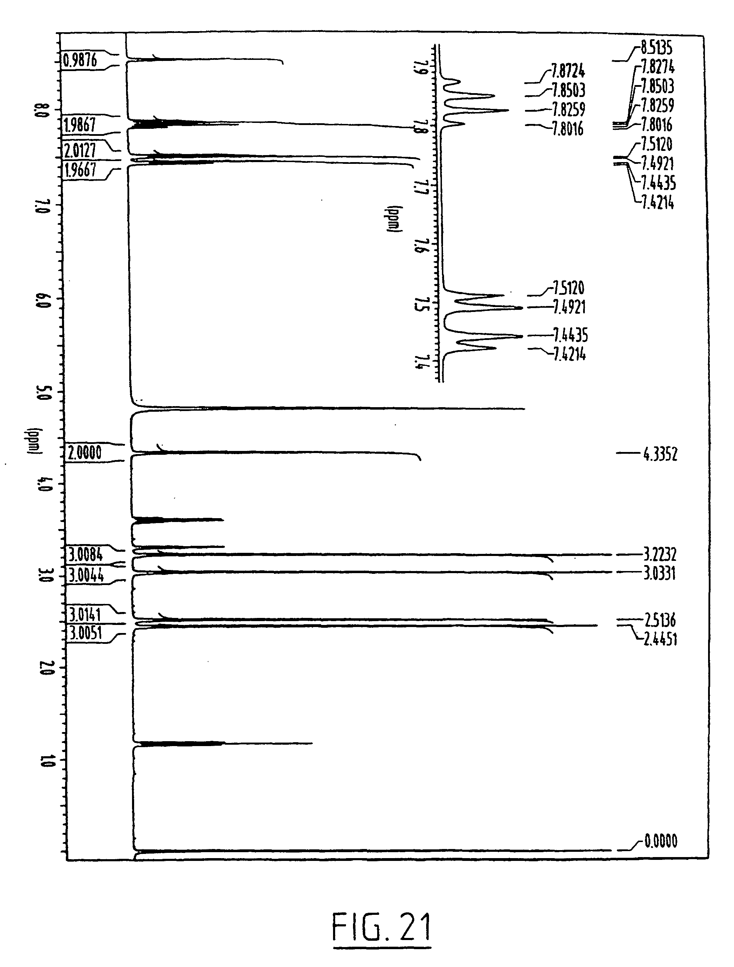

Fig. 21 shows an NMR spectrum of the zolpidem hydrochloride hemiethanolate from example 9;

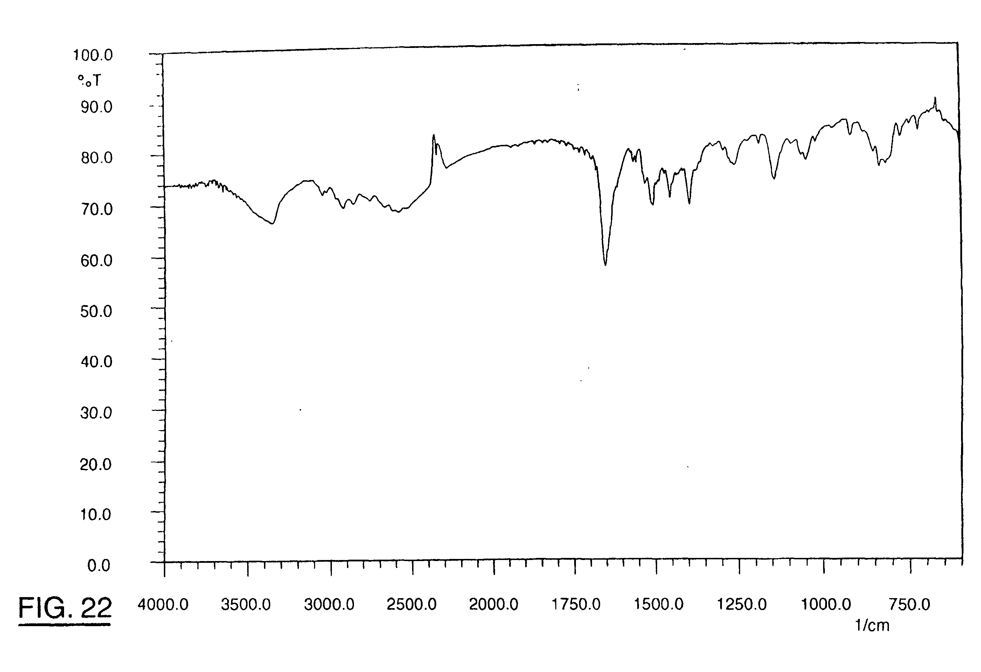

Fig. 22 shows an IR spectrum of the zolpidem hydrochloride hemiethanolate from example 9;

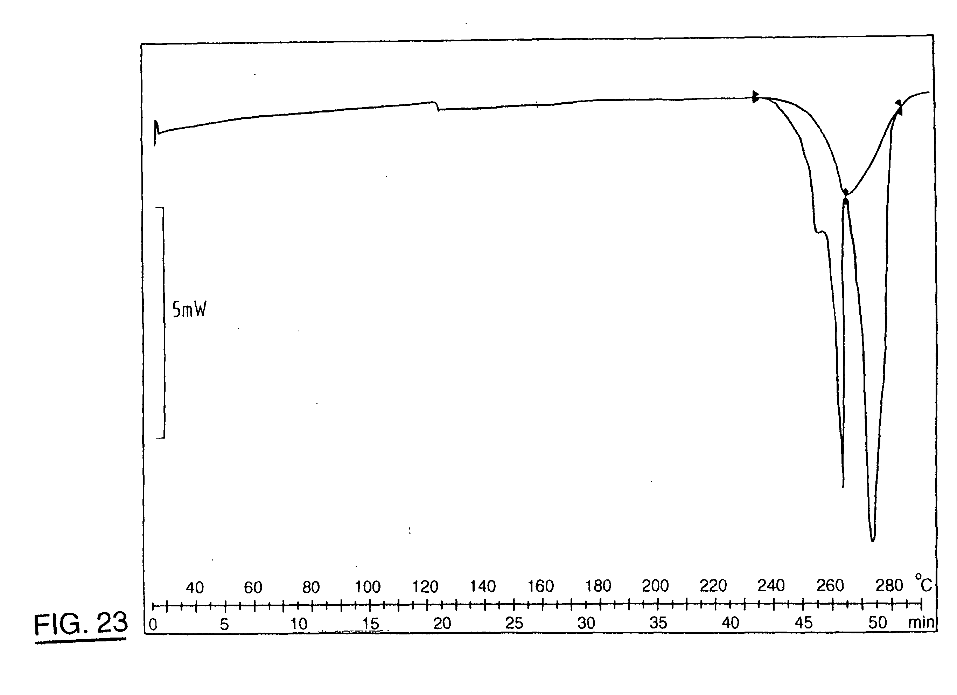

Fig. 23 shows a DSC curve of the zolpidem hydrogensulfate from example 13;

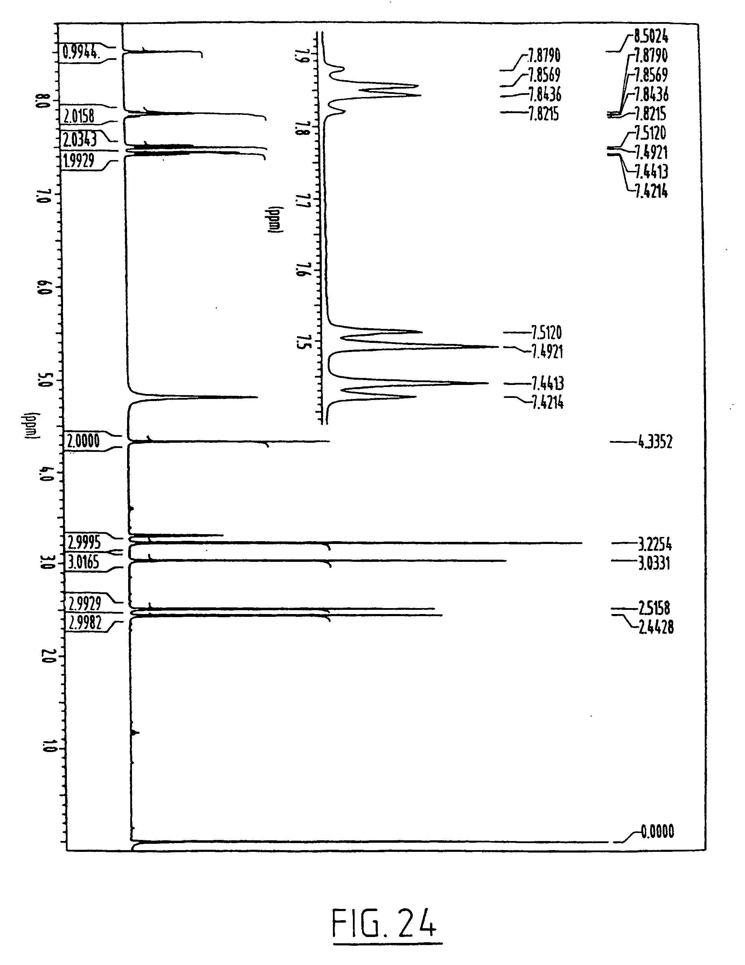

Fig. 24 shows an NMR spectrum of the zolpidem hydrogensulfate from example 13;

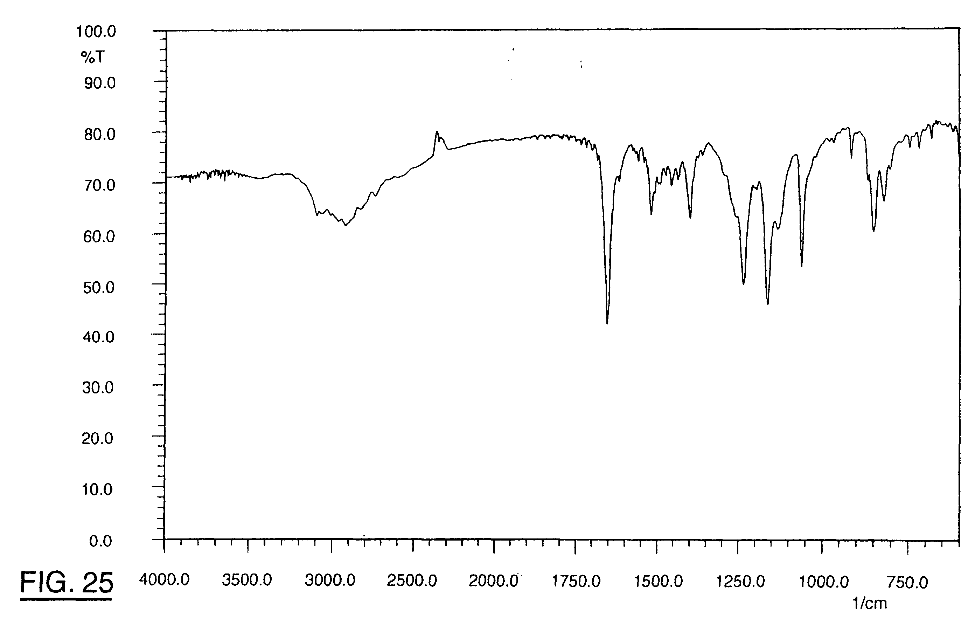

Fig. 25 shows an IR spectrum of the zolpidem hydrogensulfate from example 13;

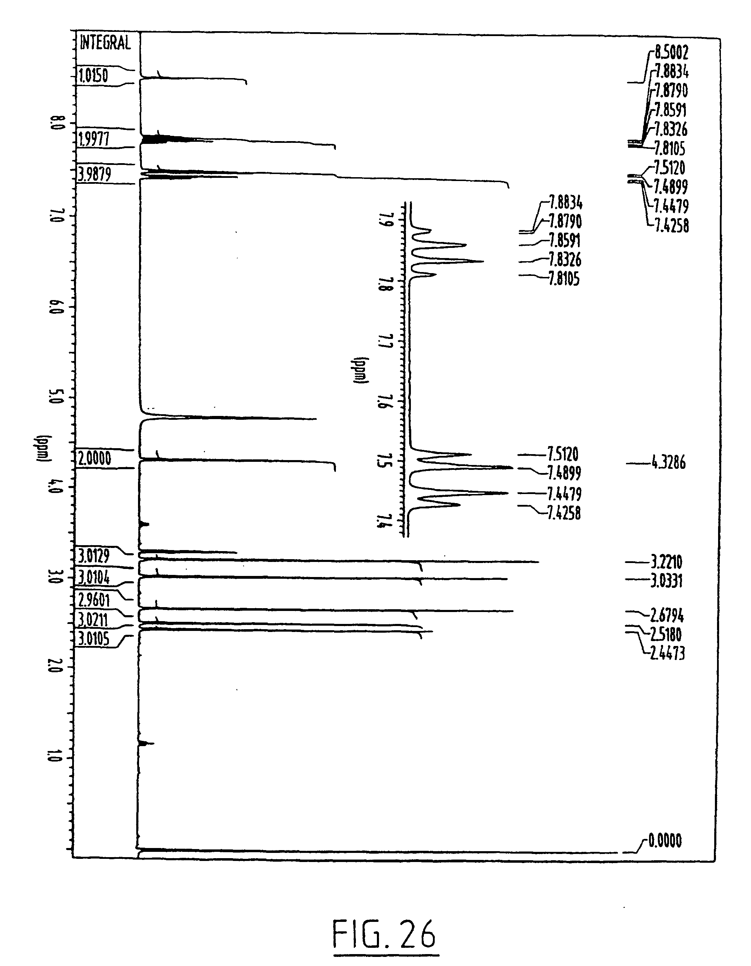

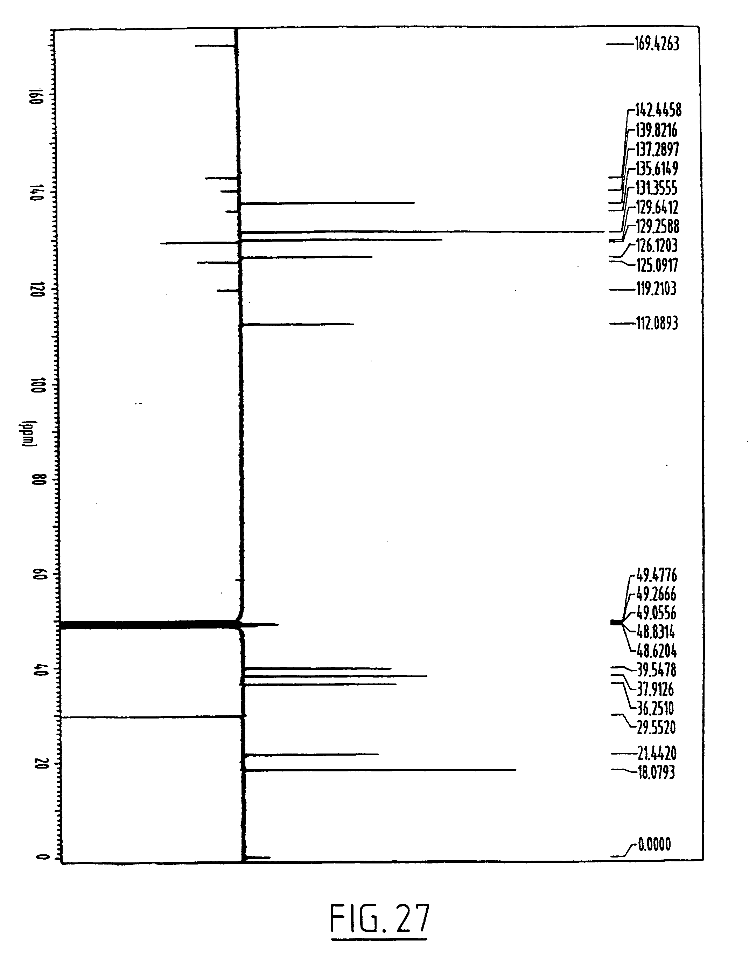

Fig. 26 and 27 show ¹H and ¹³C NMR spectra respectively of the zolpidem mesylate from example 10;

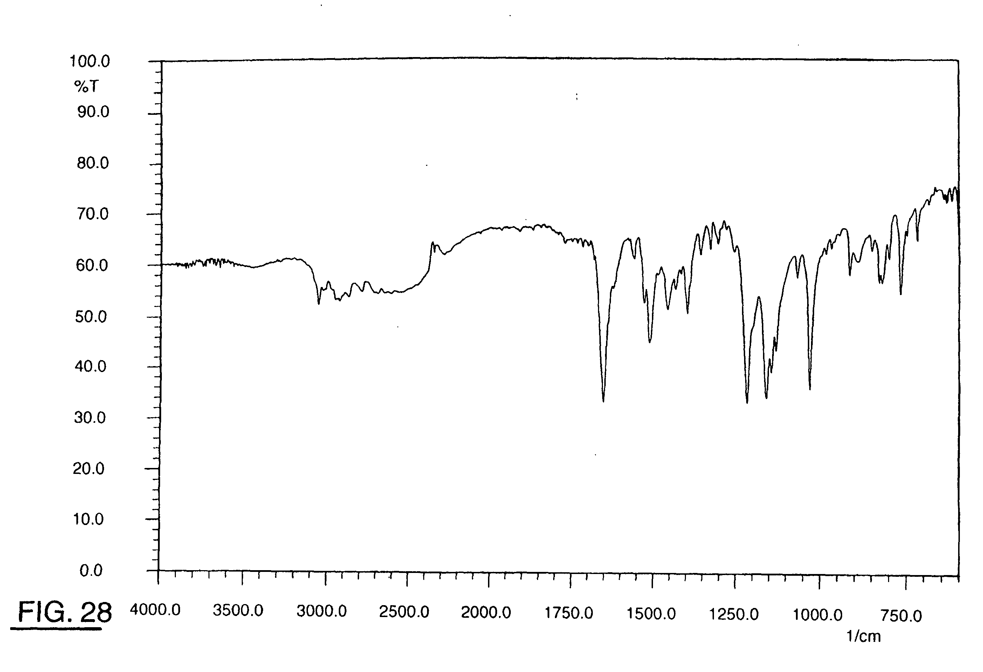

Fig. 28 shows an IR spectrum of the zolpidem mesylate from example 10;

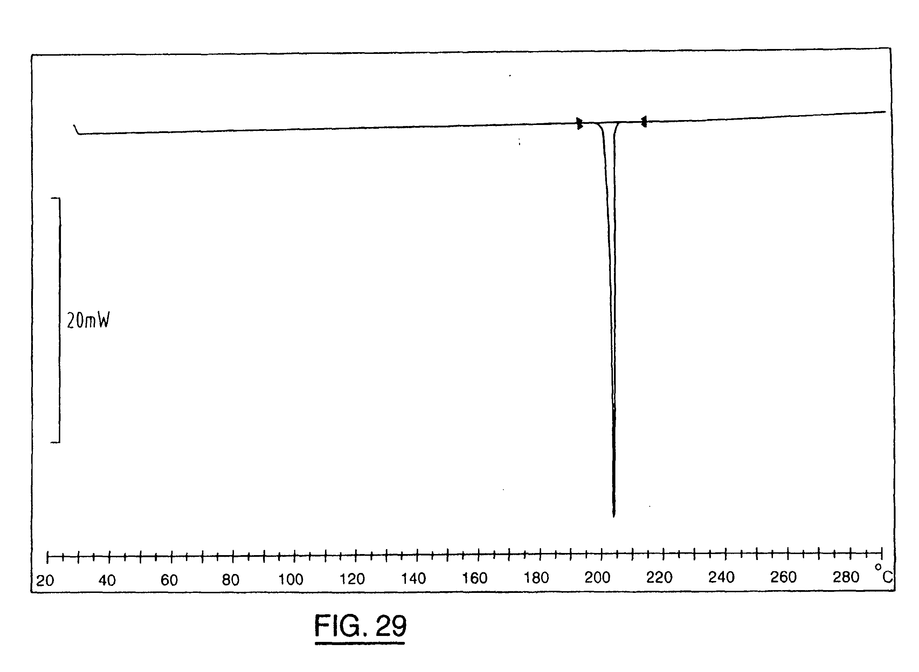

Fig. 29 shows a DSC curve of the zolpidem mesylate from example 14;

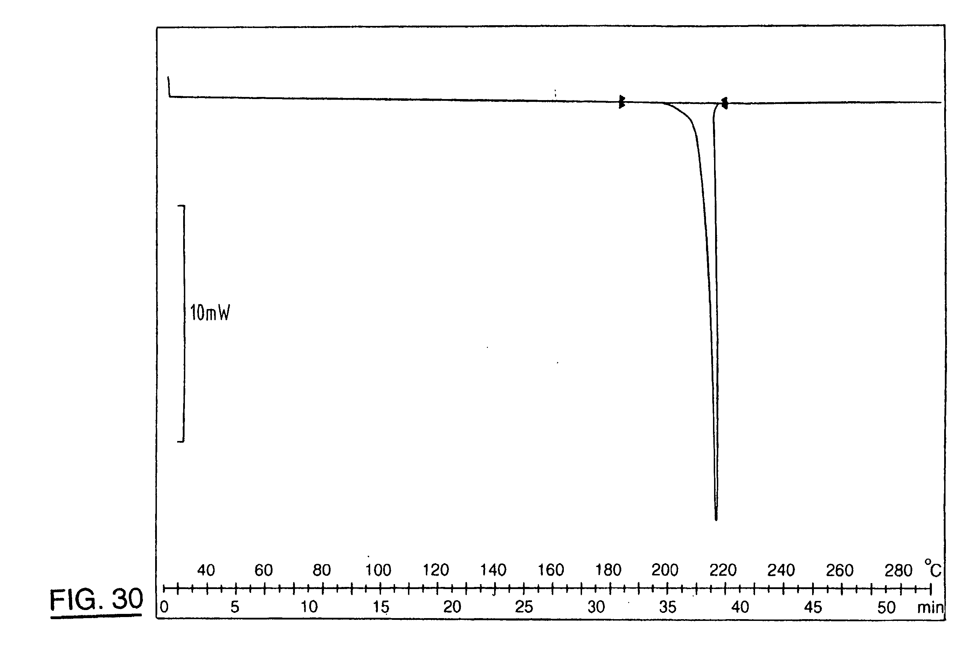

Fig. 30 shows a DSC curve of the zolpidem tosylate from example 15;

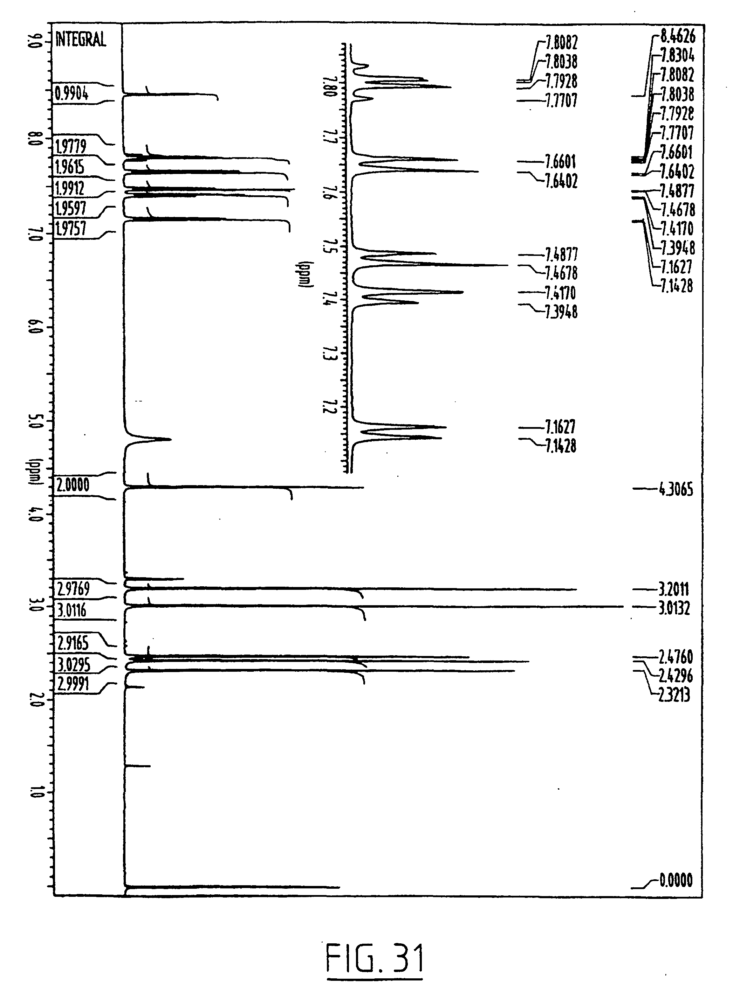

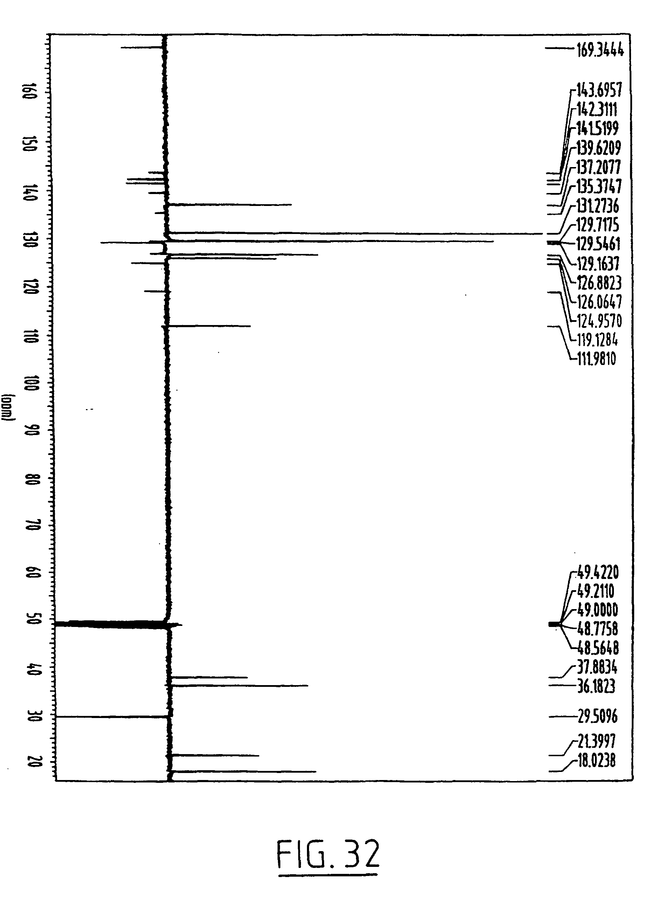

Fig. 31-32 show ¹H and ¹³C NMR spectra respectively of the zolpidem tosylate from example 15;

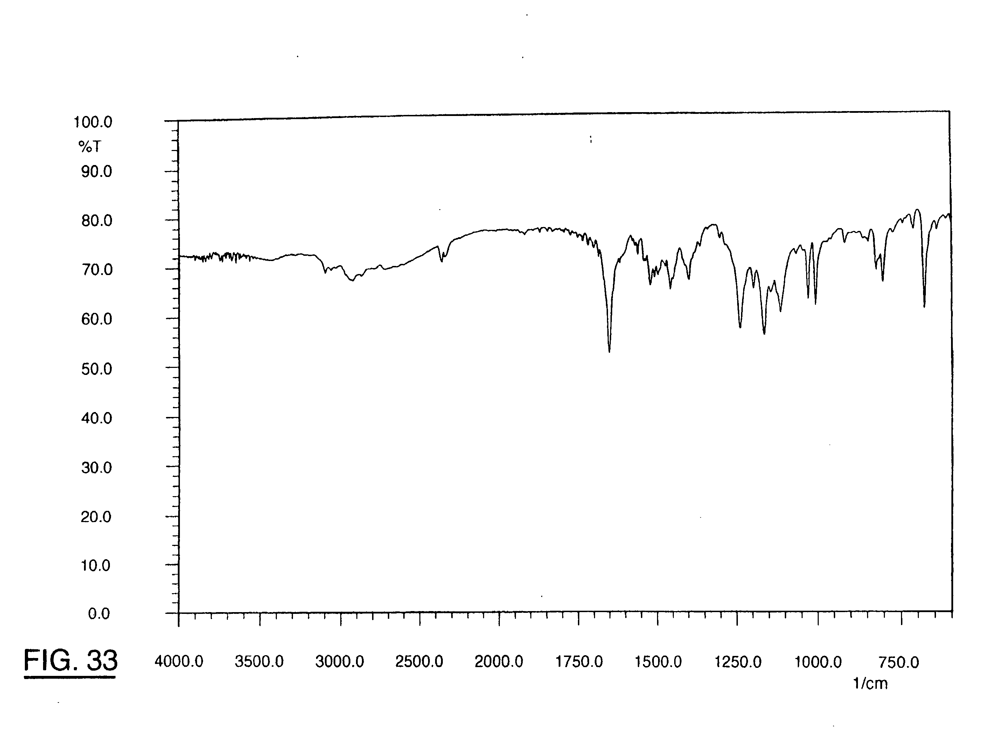

Fig. 33 shows an IR spectrum of the zolpidem tosylate from example 15;

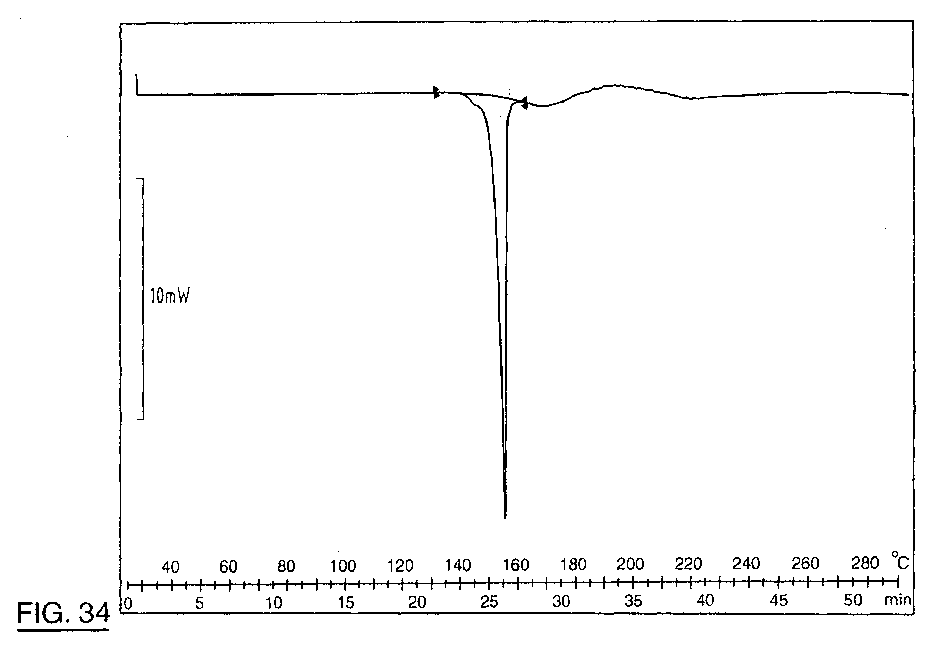

Fig. 34 shows a DSC curve of the zolpidem maleate from example 16;

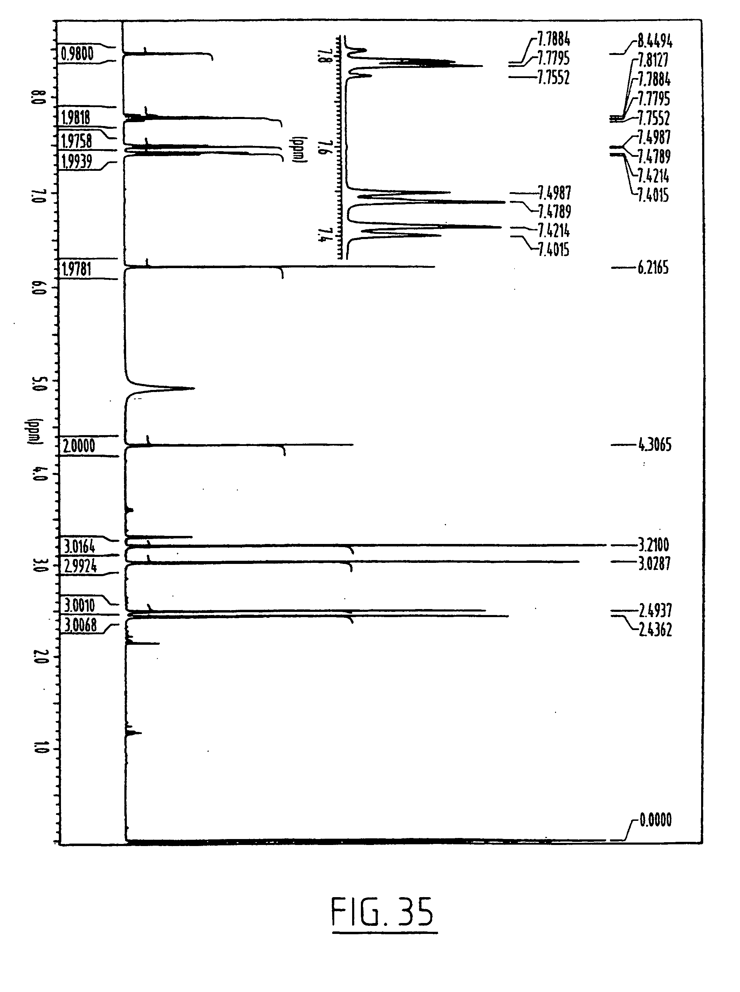

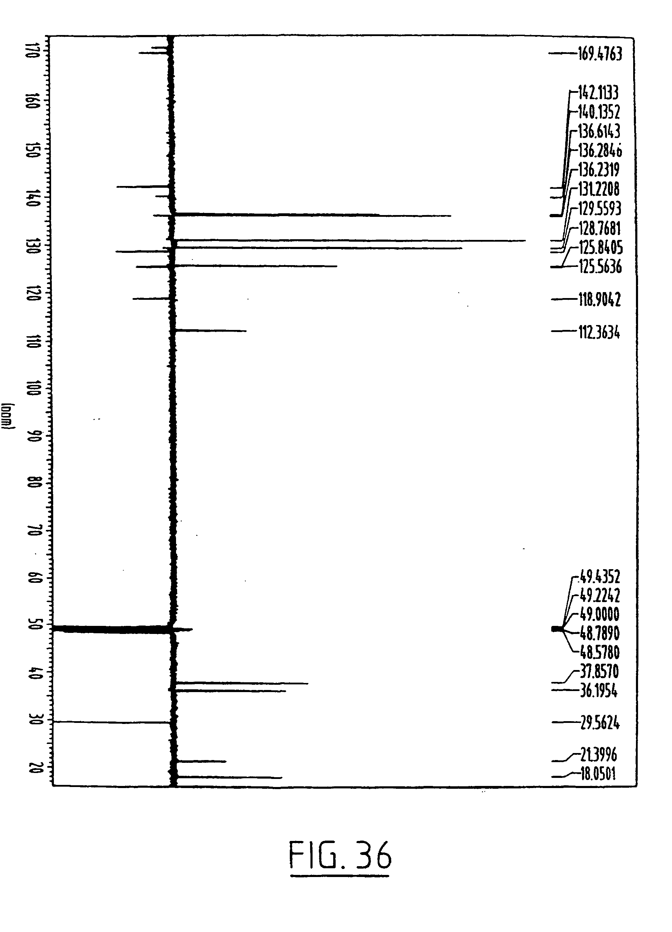

Fig. 35-36 show ¹H and ¹³C NMR spectra respectively of the zolpidem maleate from example 16;

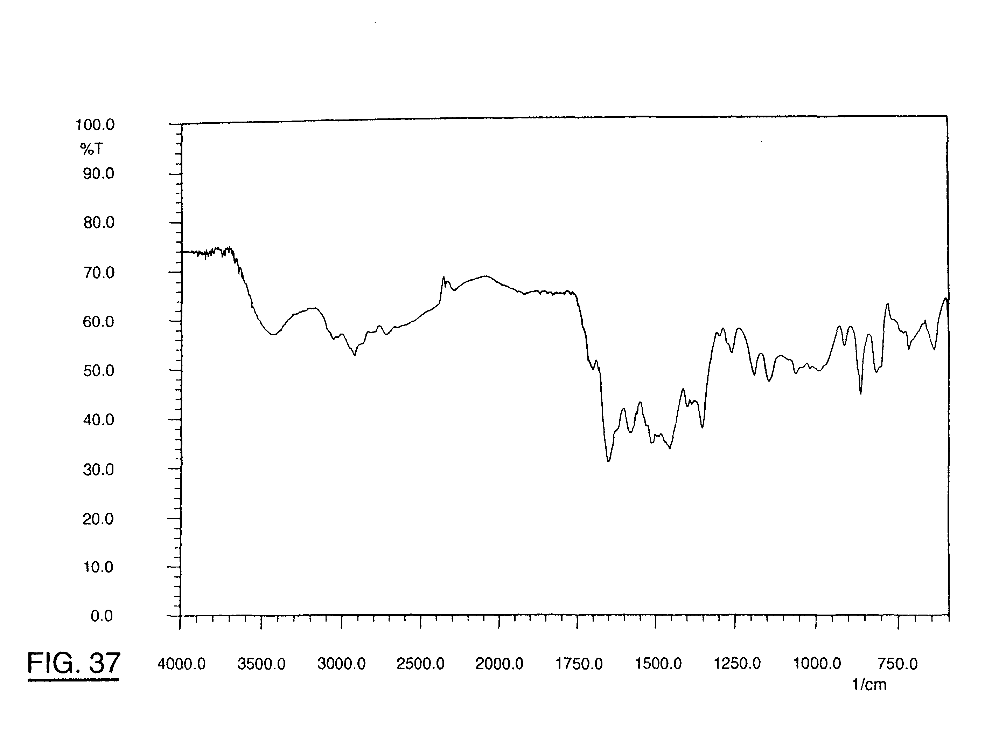

Fig. 37 shows an IR spectrum of the zolpidem maleate from example 16;

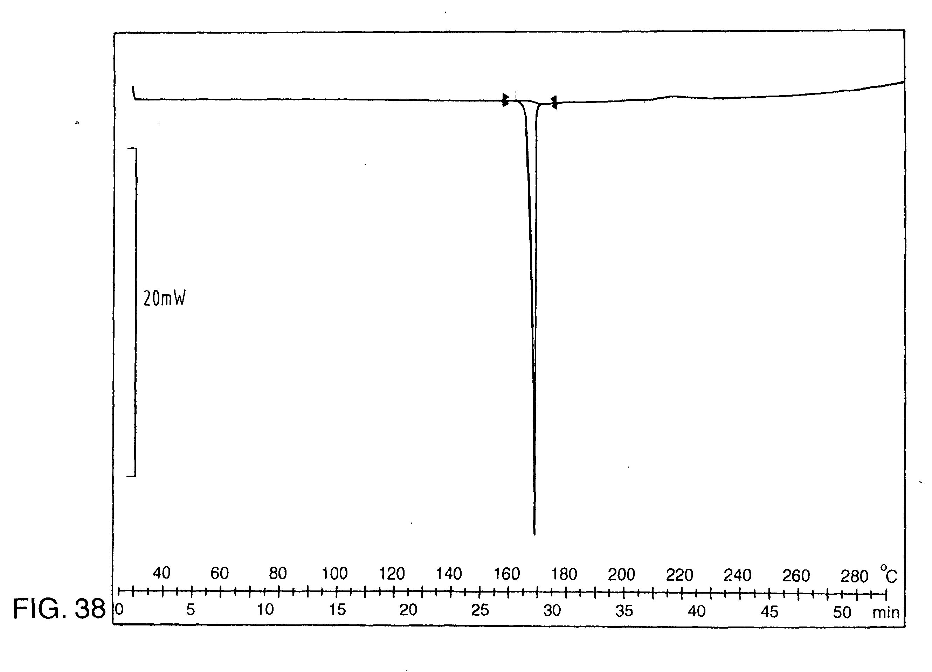

Fig. 38 shows a DSC curve of the zolpidem trifluoroacetate from example 18;

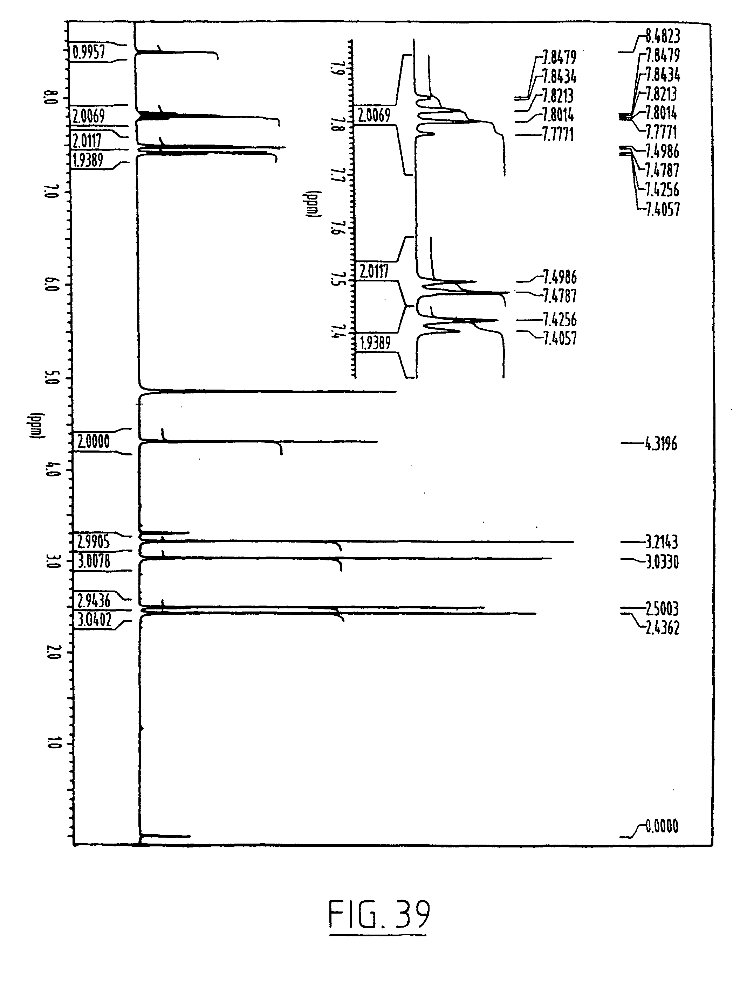

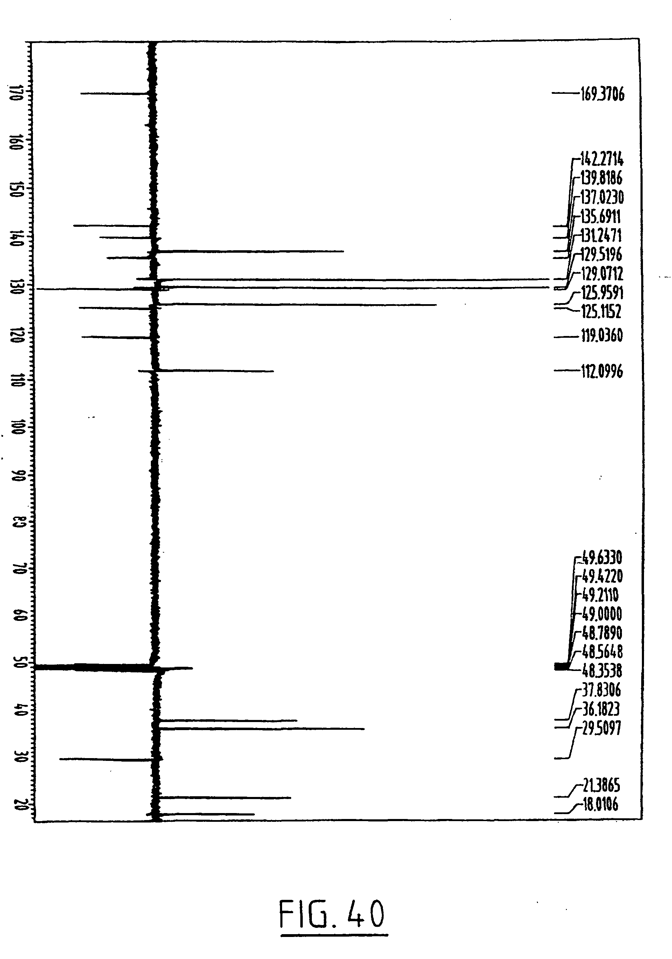

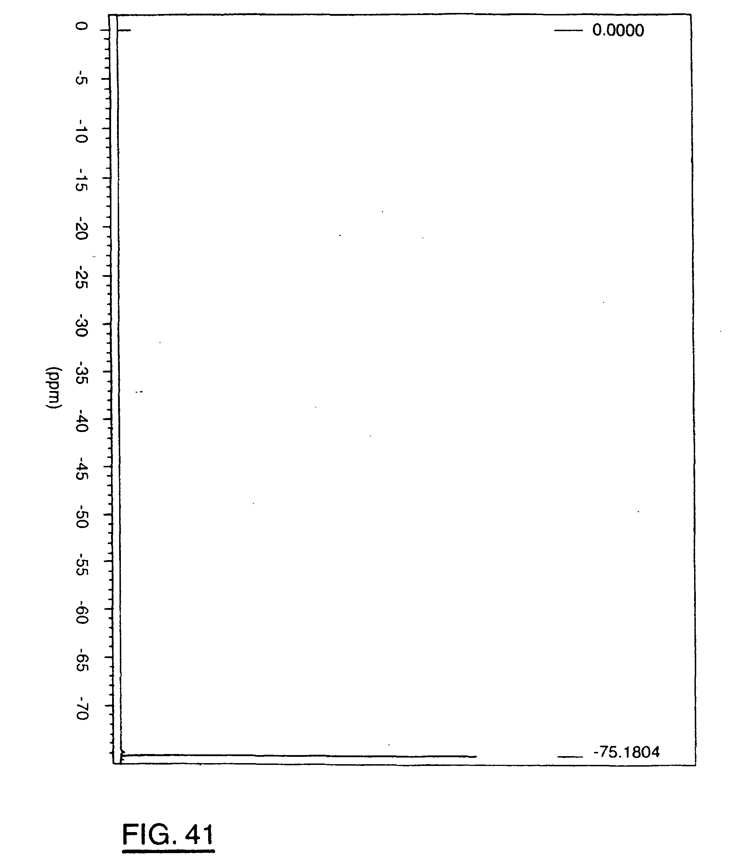

Fig. 39-41 show ¹H, ¹³C, ¹⁹F NMR spectra respectively of the zolpidem trifluoroacetate from example 18;

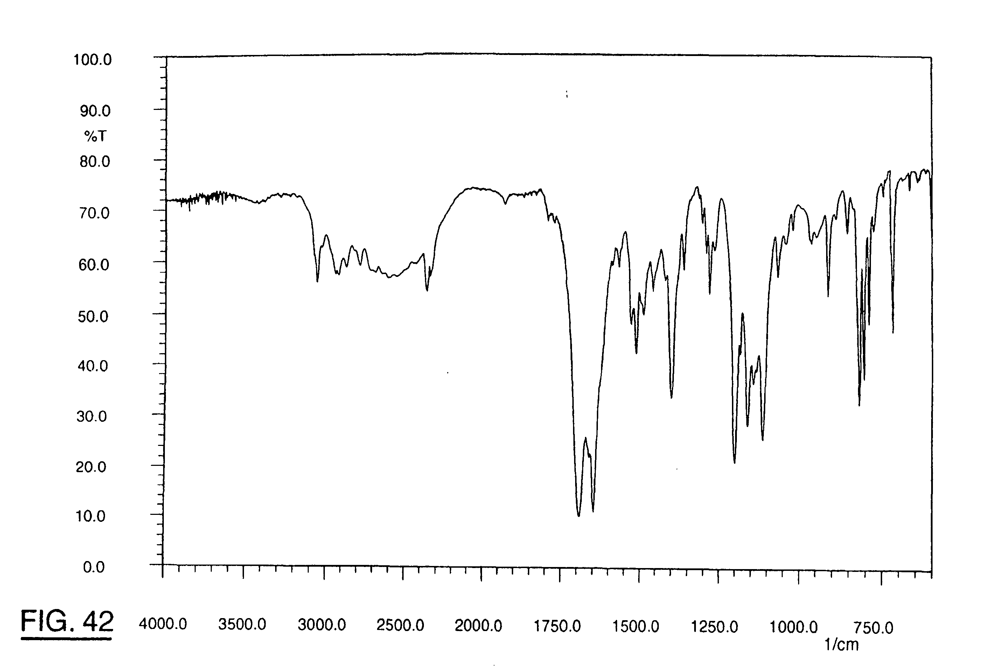

Fig. 42 shows an IR spectrum of the zolpidem trifluoroacetate from example 18; and

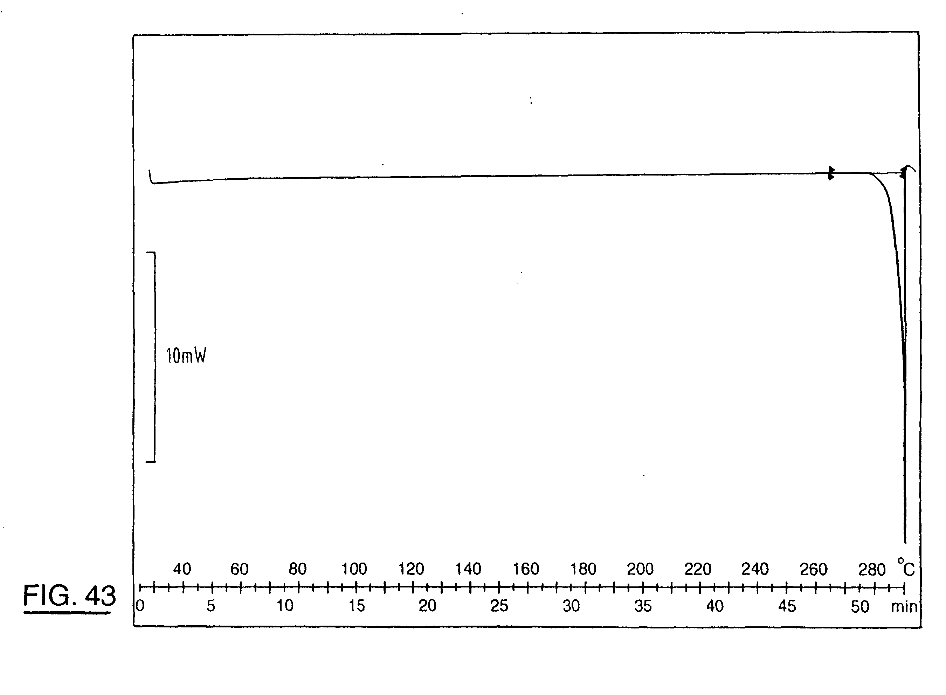

Fig. 43 shows a DSC curve of the zolpidem hydrobromide from example 19.

To determine whether a melting endotherm corresponds to the melting of a zolpidem free base fraction within the composition, a sample of the same test composition was run again but this time with zolpidem free base added and admixed therein. If the melting endotherm originally observed in the first test was larger, then the endotherm corresponded to the melting of zolpidem free base.

Alternatively, if a different melting endotherm was induced by the artificial introduction of zolpidem free base, then the original melting endotherm did not correspond to the zolpidem free base.

For clarity, "melting endotherms", as is understood in the art, are those endotherms where the operator of the DSC visually observes that the sample is melting.

### Example 1 (reference)

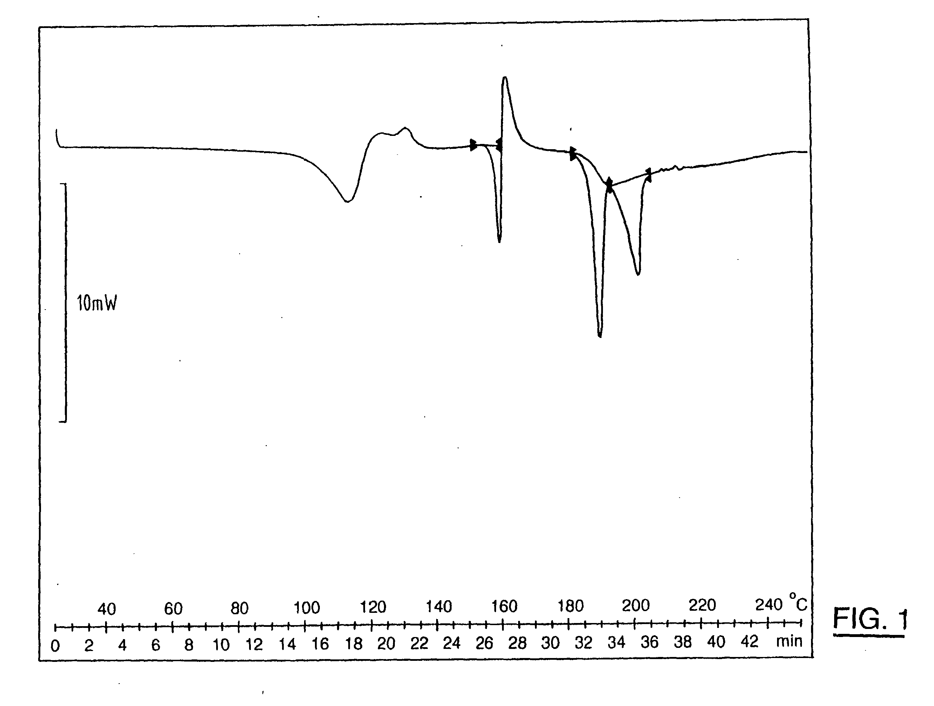

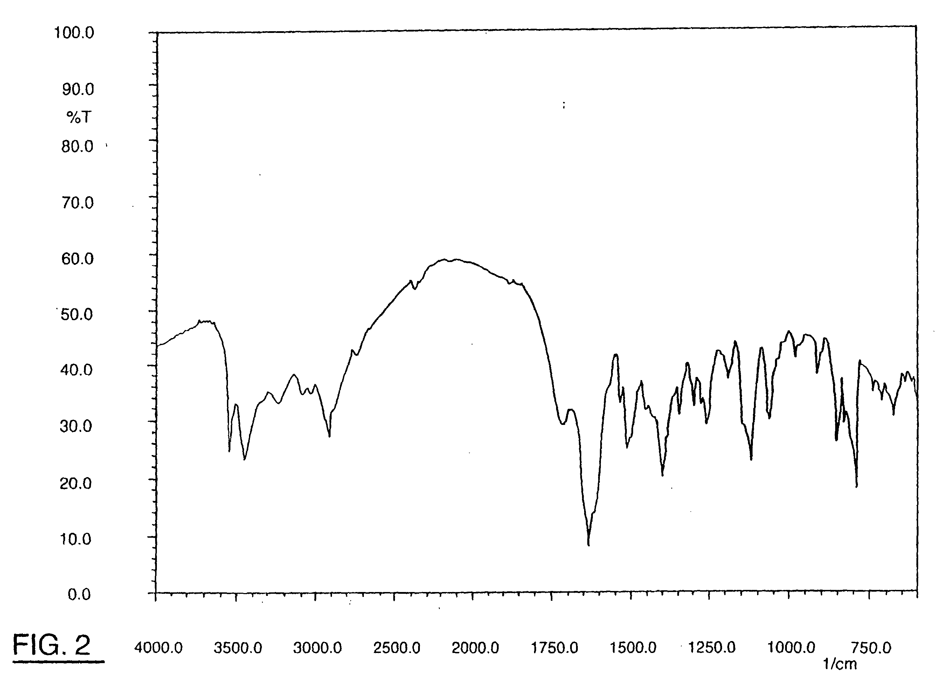

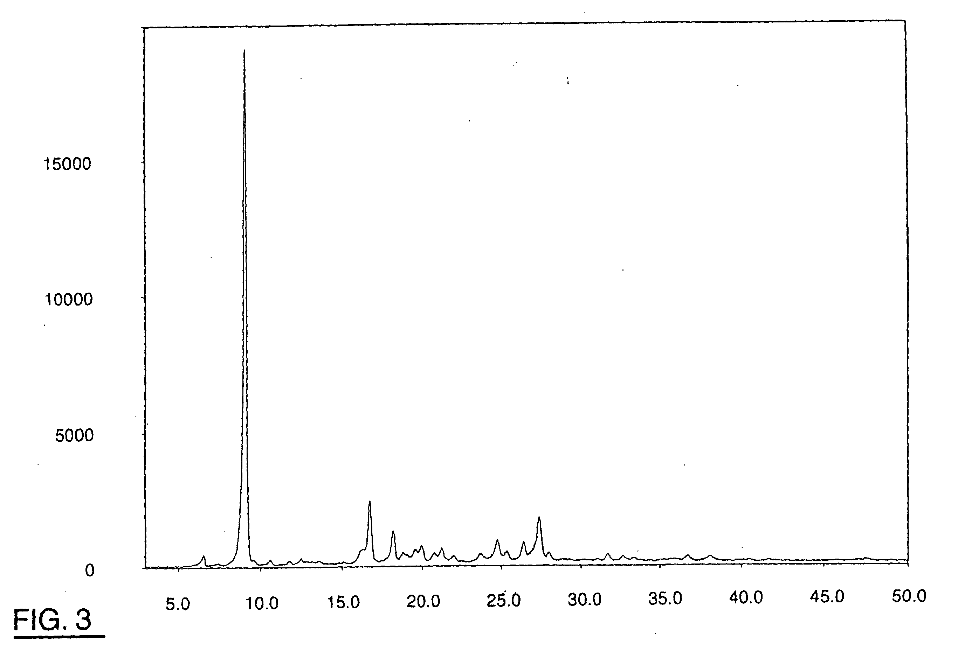

A sample of the European standard for zolpidem tartrate (CRS) was obtained and subjected to DSC analysis, IR spectroscopy, and X-ray powder diffraction using conventional and/or standard methods and techniques. Representative results for the DSC, IR and X-ray powder diffraction are set forth in figures 1, 2 and 3, respectively.

### Example 2 (reference)

A solution of 1 g (3.25 mmol) of zolpidem in 10 ml of methanol is prepared at room temperature. A second solution of 0.244 g (1.625 mmol) of tartaric acid is prepared at 50°C. Both solutions are mixed together at 50°C under stirring and the clear mixture is kept at room temperature for 6 hours. No crystals are formed. The solution is stored at 4°C overnight, and still no crystals are formed. The solution is kept at -20°C for 2 hours resulting in crystal formation. The formed crystals are filtered off and washed with ether. DSC, IR and X-ray powder diffraction analyses return the same results as figs. 1-3, indicating that the product corresponds with zolpidem tartrate CRS (European Pharmacopoeia, 3rd Ed.).

### Example 3 (crystalline zolpidem hydrogentartrate)

In a 100 ml flask, 1 g of zolpidem is dissolved in 20 ml of isopropanol under heating. In another flask, 0.244 g of tartaric acid is dissolved in 10 ml of isopropanol while heating. The hot solutions are combined under stirring and the clear solution is allowed to stand at room temperature. After 30 minutes, crystallization starts spontaneously. After standing overnight at room temperature, the formed crystals are filtered off and washed with ether. NMR Spectrum of the product reveals that it is zolpidem hydrogentartrate. Representative DSC, IR and X-ray powder diffraction graphs are shown in figures 4, 5 and 6, respectively.

### Example 4 (crystalline zolpidem hydrogentartrate)

Under stirring, 15 ml of anhydrous methanol is heated at reflux and 1 g of zolpidem and 0.244 g of tartaric acid are added while hot. The resulting solution is left to cool at room temperature overnight. The formed crystals are filtered off and dried. The product is identical to the crystalline zolpidem hydrogentartrate produced in Example 3.

### Example 5 (reference)

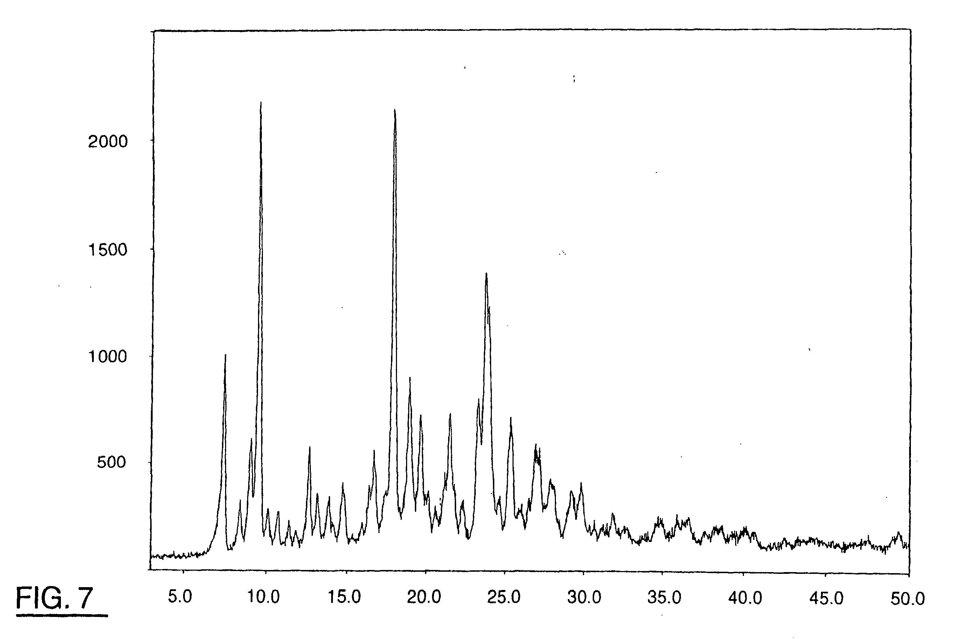

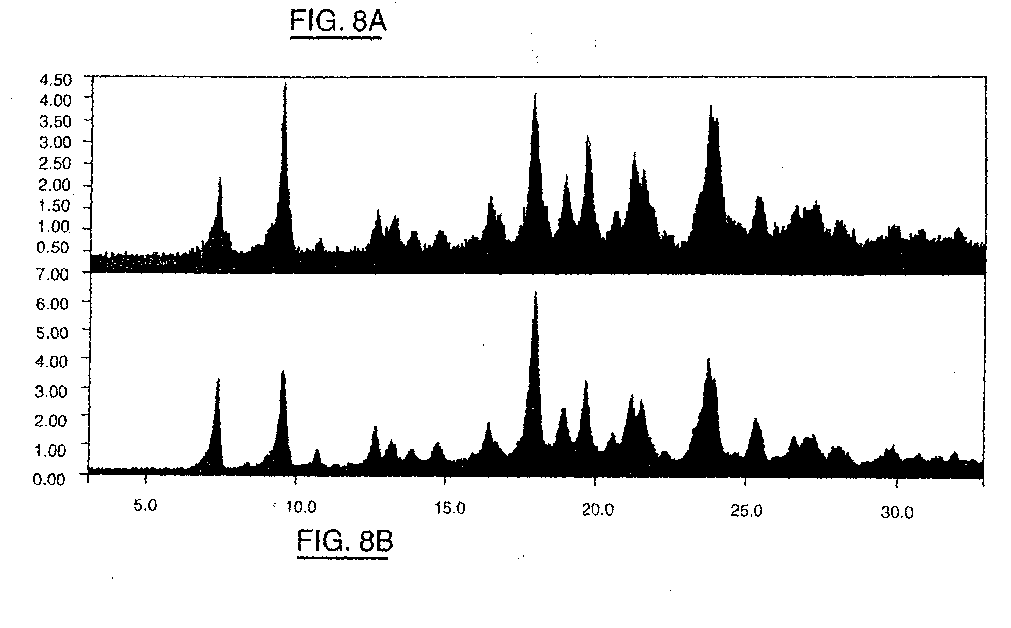

100 mg of zolpidem tartrate from Example 2 is heated over three days at 100°C. After cooling, the sample is analyzed by X-ray powder diffraction and representative results are shown in fig. 7. This heat treated product is no longer identical with zolpidem tartrate. Instead the product is identical with an artificially created equimolar mixture of zolpidem free base and zolpidem hydrogen tartrate as shown in fig. 8. Note that graph A corresponds to the 1:1 admixed product and graph B corresponds to the heat treated product. Accordingly, heating converts the zolpidem tartrate crystal into a mixture of zolpidem hydrogentartrate and zolpidem free base.

### Example 6 (amorphous zolpidem hydrogentartrate)

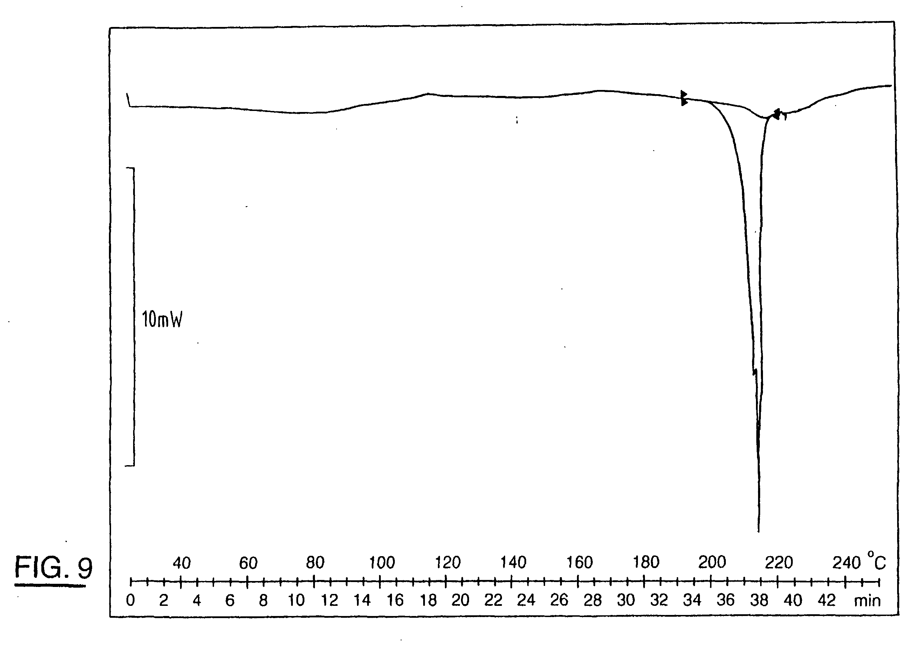

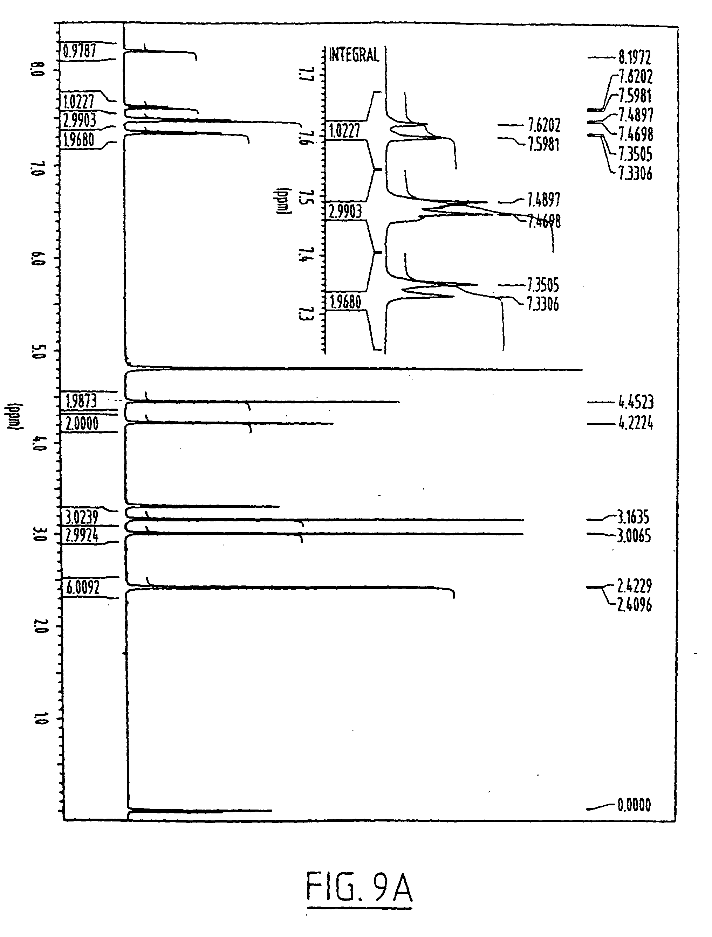

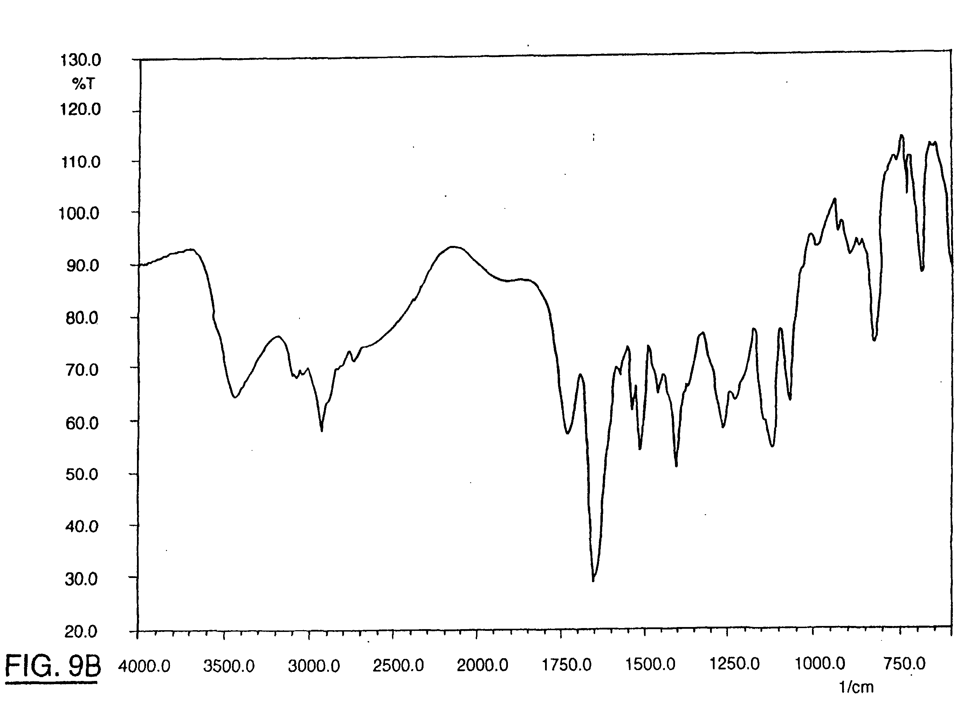

A solution of 488 mg (3.25 mmol) of tartaric acid in 100 ml of water is prepared and 1.0 g (3.25 mmol) of zolpidem is added to the solution under stirring. The resulting solution is frozen at -80°C and freeze dried. A white fluffy solid is obtained in quantitative yield and is identified as amorphous zolpidem hydrogentartrate. A representative DSC curve is shown in fig. 9.
A representative NMR-spectrum is shown in fig. 9A.
A representative infra-red spectrum is shown in fig. 9B.

| | |
|---|---|
| Yield | 1.5 g |
| NMR | Fig. 9A |
| IR | Fig. 9B |
| DSC | Fig. 9 |

### Example 7 (zolpidem hydrochloride monohydrate)

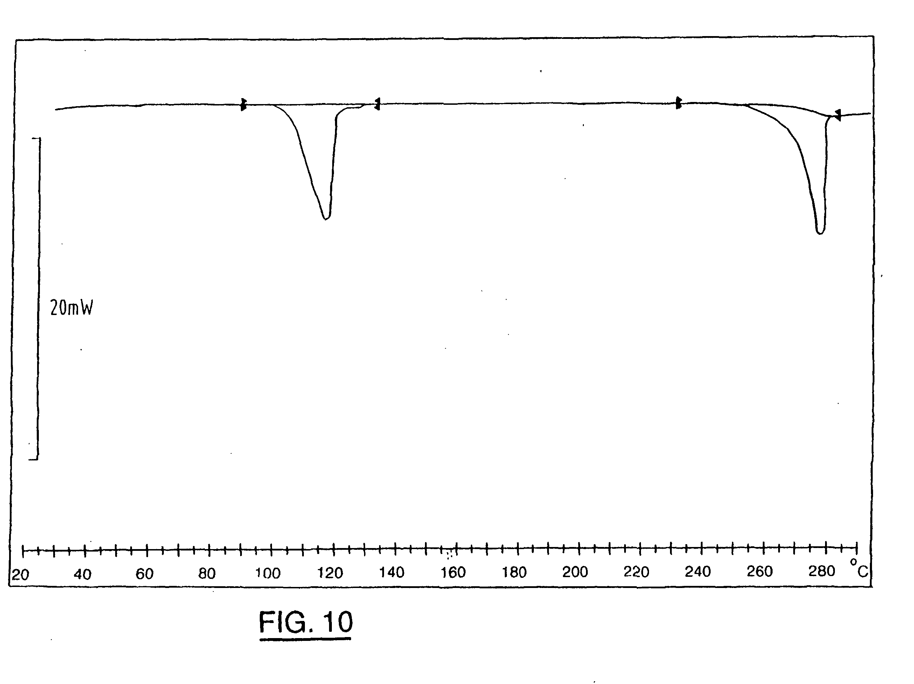

One gram of zolpidem free base is added under stirring to a solution of 0.32 g (a molar equivalent) of concentrated hydrochloric acid in 10 ml of acetone. The mixture is heated under stirring to 50°C and stirred at this temperature for 30 minutes. The mixture is allowed to cool to room temperature, the formed solid is filtered off, washed with 3 ml of acetone and dried in a vacuum oven at 40°C. The amount of water, determined by thermogravimetry (TGA), corresponds to a monohydrate. A representative DSC curve is shown in fig. 10.

| | |
|---|---|
| Yield | 1.28 g |
| M.P. | 267-277°C |
| NMR | Fig. 19 |
| IR | Fig. 19A |
| DSC | Fig. 10 |

### Example 8 (zolpidem hydrochloride anhydrate)

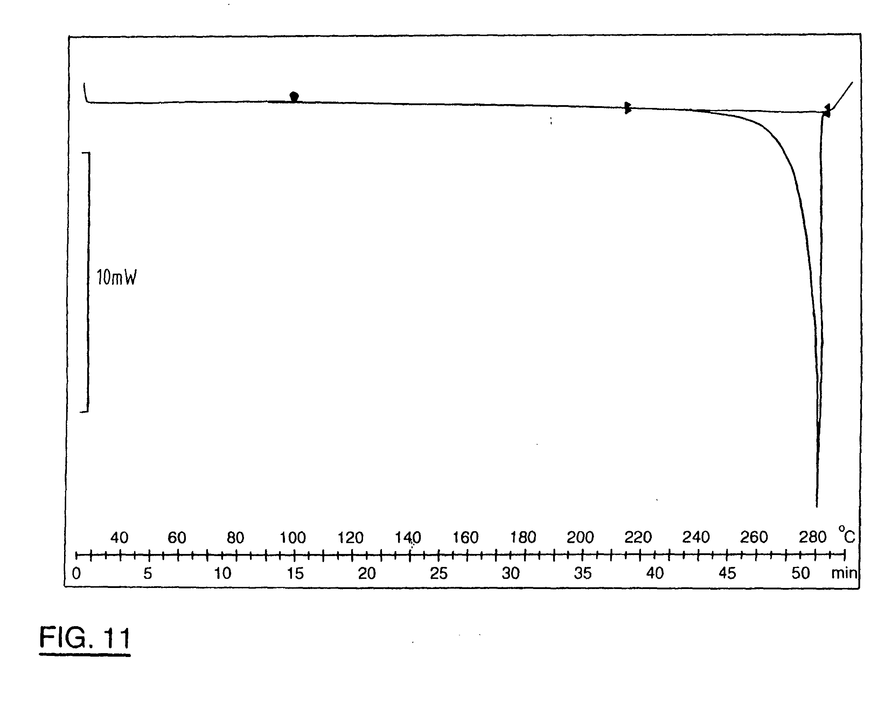

One gram of zolpidem is suspended in 35 ml of 1-butanol, 0.32 g of concentrated hydrochloric acid is added and the mixture stirred 15 minutes at room temperature. The clear solution is heated to reflux and 25 ml of a water/1-butanol mixture is distilled off. The resulting mixture is allowed to cool to room temperature and stirred at room temperature for 16 hours. The formed solid is filtered off, washed with 2 ml of 1-butanol and dried in a vacuum oven at 40°C to produce zolpidem hydrochloride anhydrate. A representative DSC curve is shown in fig. 11.

| | |
|---|---|
| Yield | 0.755 g |
| NMR | Fig. 16 |
| IR | Fig. 13-Fig.15 |
| DSC | Fig. 11 |
| MP | 268-277°C |

### Example 9 (zolpidem hydrochloride hemiethanolate)

Zolpidem free base (1.0 g) is suspended in 10 ml of ethanol and heated at 50°C until dissolution. Then, 0.32 g (molar equivalent) of concentrated hydrochloric acid is added and the solution stirred for 10 minutes. Then the bath is removed and the reaction mixture is allowed to cool to room temperature. Finally, the mixture is kept standing overnight at 4°C. The formed crystals are filtered off, washed with 3 ml of cold ethanol and dried at 40°C in a vacuum oven to produce zolpidem hydrochloride hemiethanolate.

| | |
|---|---|
| Yield | 0.47 g |
| MP | > 240°C (degradation before melting) |
| NMR | Fig. 21 |
| IR | Fig. 22 |
| DSC | Fig. 20 |

### Example 10 (zolpidem methanesulfonate)

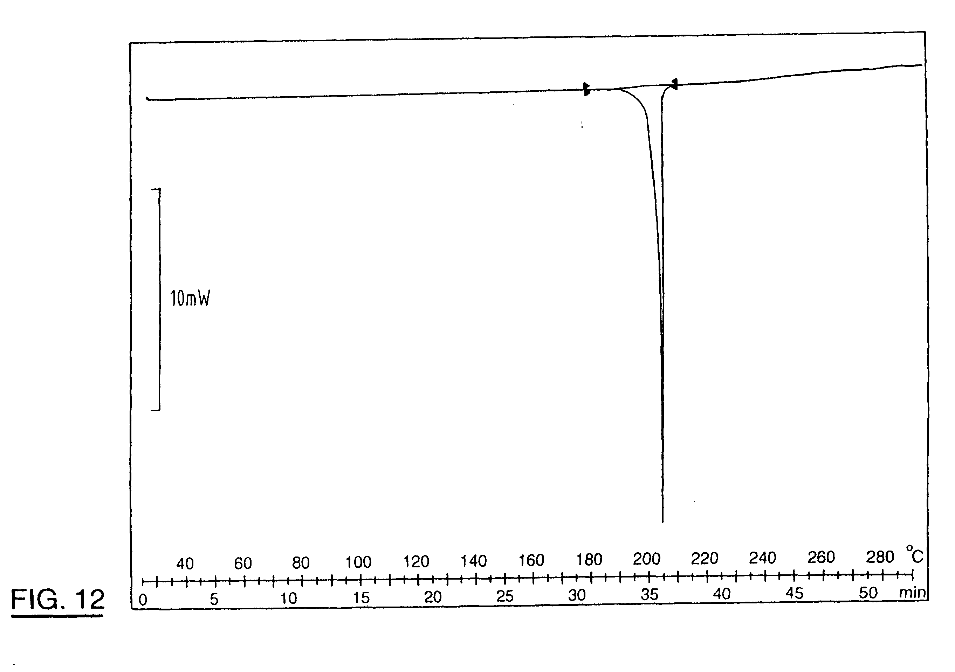

One gram of zolpidem free base is dissolved in 10 ml of ethanol at 50°C. Then 0.313 g (a molar equivalent) of methane sulfonic acid is added at this temperature, the mixture is stirred for 5 minutes and evaporated to dryness under reduced pressure. The residue is treated with 10 ml of acetone, the remaining solid is filtered off and washed with 5 ml of acetone and dried in a vacuum oven at 40°C to yield zolpidem methanesulfonate. A representative DSC curve is shown in fig. 12.

| | |
|---|---|
| Yield | 1.22 g |
| NMR | Fig. 26, Fig. 27 |
| IR | Fig. 28 |
| DSC | Fig. 12 |
| MP | 198-200°C |

### Example 11 (zolpidem hydrochloride from CH₂Cl₂) and HCl gas)

### Procedure:

1 g zolpidem was dissolved in 10 ml dry dichloromethane. HCl gas was bubbled through for 30 min. The solid that was formed was filtered off and washed with 2 ml dry dichloromethane. Dried in vacuum oven at 40°C.

| | |
|---|---|
| DSC | Fig. 17 |

### Example 12 (zolpidem hydrochloride monohydrate)

### Procedure:

At room temperature, 5 g zolpidem was added to a solution of 1.6 g concentrated HCl in 50 ml acetone, stirred for 10 minutes at room temperature. Heated to 50°C for 30 minutes. Allowed to cool to room temperature, the solid was filtered off and washed with 10 ml acetone. Dried in a vacuum oven at 50°C.

| | |
|---|---|
| Yield | 5.15 g |
| MP | 267-277°C (degradation before melting) |
| DSC | Fig. 18 |

### Example 13 (zolpidem hydrogen sulfate)

### Procedure:

1 g zolpidem was dissolved in 10 ml ethanol at 50°C. 0.336 g Concentrated sulfuric acid was added. After adding a white solid was formed. The suspension was allowed to cool to room temperature, the solid filtered off and washed with 3 ml cold ethanol, dried in a vacuum oven at 50°C for 1 hour.

| | |
|---|---|
| Yield | 1.20 g |
| MP | >235-237°C (degradation before melting) |
| NMR | Fig. 24 |
| IR | Fig. 25 |
| DSC | Fig. 23 |

### Example 14 (zolpidem mesylate crystallized from acetone)

### Procedure:

At room temperature, 5 g zolpidem was added to a solution of 1.56 g Methane sulfonic acid in 50 ml acetone, stirred for 10 minutes at room temperature. Heated to 50°C for 30 minutes. Allowed to cool to room temperature, filtered off solid, washed with 3 x 4 ml acetone, dried in a vacuum oven at 25°C.

| | |
|---|---|
| Yield | 6.1 g |
| DSC | Fig. 29 |

### Example 15 (Zolpidem tosylate)

### Procedure:

1 g zolpidem was suspended in 5 ml acetone at 50°C. 0.62 g p-toluene sulfonic acid was dissolved in 5 ml acetone and added to the hot suspension. A clear solution was obtained after 5 min. The solution was allowed to cool to room temperature and set aside at 4°C. After 1 hour a solid was formed. Filtered off, washed with 3 ml acetone, dried in a vacuum oven at 40°C.

| | |
|---|---|
| Yield | 1.5 mg |
| NMR | Fig. 31 - Fig. 32 |
| IR | Fig. 33 |
| DSC | Fig. 30 |
| MP | 209-212°C |

### Example 16 (zolpidem maleate)

### Procedure:

1 g zolpidem was dissolved in 10 ml ethanol at 50°C. 0.38 g.Maleic acid was added. The resulting solution was stirred for 10 minutes and allowed to cool to room temperature. Set aside at 4°C for 2 hours. No solid, solvent evaporated. To residue added 10 ml acetone. Again solvent evaporated leaving a solid.

| | |
|---|---|
| Yield | 1.34 g |
| NMR | Fig. 35, 36 |
| IR | Fig. 37 |
| DSC | Fig. 34 |

### Example 17 (Zolpidem trifluoroacetate)

### Procedure:

1 g zolpidem was dissolved in 10 ml ethanol at 50°C. 0.37 g trifluoroacetic acid was added. The resulting solution was stirred for 10 minutes and allowed to cool to room temperature. Set aside at 20°C for 2 hours. No solid, Set aside at 4°C for 1 night. No solid, set aside at -20°C. No solid, evaporated off solvent and added 10 ml acetone. Filtered off solid, washed with 3 ml acetone.

| | |
|---|---|
| Yield | 1.1 g |
| NMR | Fig. 39-41 |
| MP | 163-165°C |
| IR | Fig. 42 |
| DSC | Fig. 38 |

### Example 18 (Zolpidem hydrobromide)

### Procedure:

5 g zolpidem was suspended in 50 ml acetone and heated to 50°C. 2.75 g HBr-solution in water (48%) was added and the suspension was stirred for 1 hour at 50°C. The reaction mixture was allowed to cool to room temperature and the solid was filtered off. The solid was washed with 2 x 3 ml acetone and dried in a vacuum oven at 30°C for 16 hours.

| | |
|---|---|
| Yield | 6.05 g |
| MP | 280°C (degradation before melting) |
| DSC | Fig. 43 |

Throughout this specification, the wording "tartaric acid" means naturally occurring L(+) tartaric acid. However, mutatis mutandis, the described procedures are useful also for the production of the corresponding salts of zolpidem with D- or DL-tartaric acid.

### Determination of solubility

For each investigated salt form the values for C_{S,av} are presented in the table below. The value for C_{S,salt} represents the solubility of each salt form in water at 25°C. Though the solubility is defined by the salt form, C_{S,av} can be used if the solubility in mg Zolpidem/ml, instead of mg active substance/ml is desired.

**Table:**

| Values for C_{S,av} and C_{S,salt} | | |
|---|---|---|
| sample | C_{S,av} [mg zolpidem/ml] | C_{S,salt} [mg salt/ml] |
| zolpidem free base | 0.13 | 0.13 |
| zolpidem tartrate | 15.10 | 18.78 |
| zolpidem hydrogen tartrate | 13.35 | 19.85 |
| zolpidem sulfate | 114.02 | 150.40 |
| zolpidem hydrochloride monohydrate | 93.40 | 109.96 |
| zolpidem mesylate | 329.13 | 432.03 |
| zolpidem trifluoroacetate | 20.92 | 28.69 |
| zolpidem tosylate | 13.77 | 22.29 |

From the table it can be seen that Zolpidem free base is almost insoluble in water. The solubilities of known zolpidem tartrate, zolpidem hydrogentartrate, zolpidem hemitartrate, zolpidem tartrate and zolpidem tosylate do not differ a lot from each other. The solubility of Zolpidem trifluoroacetate is slightly higher. Zolpidem hydrochloric hydrate, Zolpidem sulphate and Zolpidem mesylate have a significant higher solubility. The highest solubility is obtained with Zolpidem mesylate.

The invention is not limited to the above description; the requested rights are rather determined by the following claims.

## Claims

1. A zolpidem salt, which is selected from the group of zolpidem hydrochloride, zolpidem hydrochloride monohydrate, zolpidem hydrochloride ethanolate, zolpidem methanesulfonate (mesylate), zolpidem tosylate, zolpidem maleate, zolpidem acetate, zolpidem triofluoroacetate, zolpidem hydrobromide, zolpidem fumarate, zolpidem hydrogen sulfate and zolpidem hydrogen tartrate.

2. The zolpidem salt according to claim 1, wherein the molar ratio of zolpidem moiety to anion is within the range from about 0.9-1.35:1.

3. The zolpidem salt according to claims 1 or 2, wherein said molar ratio of zolpidem moiety to anion is about 1:1.

4. The zolpidem salt according to any of the preceding claims, wherein said salt form exhibits a single melting endotherm when heated.

5. The zolpidem salt according to any of the preceding claims, wherein the solid-phase is amorphous.

6. The zolpidem salt according to any of the preceding claims wherein the solid-phase is crystalline.

7. The zolpidem salt according to any of the preceding claims, wherein said salt is substantially free of bound water and organic solvent.

8. The zolpidem salt according to any of the preceding claims wherein said salt is zolpidem hydrogentartrate, zolpidem hydrochloride, zolpidem mesylate, zolpidem tosylate, and zolpidem hydrogen sulfate.

9. The zolpidem salt according to claim 8, which is zolpidem hydrogentartrate.

10. The zolpidem salt according to claim 9, wherein said salt form is crystalline having a melting point at about (203-204°C) and/or exhibiting a single melting exotherm under DSC analysis using 5°C/min of about (209-211°C).

11. The zolpidem salt according to any of the preceding claims, selected from the group consisting of amorphous zolpidem hydrogentartrate, amorphous zolpidem tartrate, crystalline zolpidem hydrochloride anhydrate, amorphous zolpidem hydrochloride, crystalline hydrochloride zolpidem monohydrate, crystalline zolpidem hydrochloride anhydrate, crystalline zolpidem hydrochloride hemi-ethanoate, crystalline zolpidem hydrogen sulfate, and crystalline zolpidem mesylate.

12. A pharmaceutical composition comprising a pharmaceutically effective concentration of a zolpidem salt form as claimed in any of the preceding claims 1-11, and a pharmaceutically acceptable carrier or diluent.

13. The composition according to claim 12, wherein said composition is a solid dosage form.

14. The composition according to claim 13, wherein said zolpidem salt is contained in an amount within the range from about 5 mg to 50 mg per unit dose.

15. A pharmaceutical composition comprising a pharmaceutically acceptable liquid carrier and having dissolved therein an effective sleep inducing amount of the zolpidem salt according to any of the claims 1-11.

16. The liquid pharmaceutical composition according to claim 15, wherein said liquid carrier is an aqueous solution.

17. A method, which comprises dissolving a zolpidem salt form according to any of the claims 1-11 in a solvent.

18. The method according to claim 17, wherein said solvent is a pharmaceutically acceptable carrier or diluent.

19. A process which comprises:
combining zolpidem hydrogentartrate of claim 8 with a zolpidem free base in molar ratio of 1:1 in methanol to form a methanolic solution having a temperature of at least 50°C; and cooling said methanolic solution to precipitate solid zolpidem tartrate.

20. A process for providing amorphous zolpidem tartrate comprising the steps of mixing in a solvent zolpidem hydrogentartrate of claim 8 with zolpidem free base in a molar ratio of about 1:1 and separating the solid product by drying.

21. A process for providing a zolpidem salt of claims 1-11, comprising the steps of mixing together in a solvent under reactive conditions zolpidem free base and an acid, with the proviso that methanol is not used as solvent when preparing zolpidem tartrate.

22. A zolpidem salt obtainable according claim 21, which zolpidem salt has sufficient physical stability that upon heating from about 20°C to about 250°C at a rate of 5°C/minute, does not exhibit a melting endotherm that corresponds to zolpidem free base.

23. Use of a zolpidem salt according to any of the claims 1-11, 22 for preparing a medicament.

24. Use of a zolpidem salt according to any of the claims 1-11, 22 for the manufacture of a medicament for treating sleep disorders.

25. Use of a first zolpidem salt according to any of the claims 1-11, 22 in the manufacture of a separate zolpidem salt.

## Patentansprüche

1. Zolpidem-Salz, das aus der Gruppe von Zolpidemhydrochlorid, Zolpidemhydrochlorid-Monohydrat, Zolpidemhydrochlorid-Ethanolat, Zolpidemmethansulfonat (-mesylat), Zolpidemtosylat, Zolpidemmaleat, Zolpidemacetat, Zolpidemtrifluoracetat, Zolpidemhydrobromid, Zolpidemfumarat, Zolpidemhydrogensulfat und Zolpidemhydrogentartrat ausgewählt ist.

2. Zolpidem-Salz nach Anspruch 1, in dem das Molverhältnis der Zolpidem-Einheit zum Anion im Bereich von etwa 0,9 - 1,35 : 1 liegt.

3. Zolpidem-Salz nach Anspruch 1 oder 2, in dem das Molverhältnis der Zolpidem-Einheit zum Anion etwa 1 : 1 beträgt.

4. Zolpidem-Salz nach irgendeinem der vorangehenden Ansprüche, in dem die Salz-Form eine einzige Schmelzendotherme zeigt, wenn sie erwärmt wird.

5. Zolpidem-Salz nach irgendeinem der vorangehenden Ansprüche, in dem die Festphase amorph ist.

6. Zolpidem-Salz nach irgendeinem der vorangehenden Ansprüche, in dem die Festphase kristallin ist.

7. Zolpidem-Salz nach irgendeinem der vorangehenden Ansprüche, in dem das Salz im Wesentlichen frei von gebundenem Wasser und organischem Lösungsmittel ist.

8. Zolpidem-Salz nach irgendeinem der vorangehenden Ansprüche, in dem das Salz Zolpidemhydrogentartrat, Zolpidemhydrochlorid, Zolpidemmesylat, Zolpidemtosylat und Zolpidemhydrogensulfat ist.

9. Zolpidem-Salz nach Anspruch 8, das Zolpidemhydrogentartrat ist.

10. Zolpidem-Salz nach Anspruch 9, in dem die Salz-Form kristallin mit einem Schmelzpunkt bei etwa (203 - 204 °C) ist und/oder eine einzige Schmelzexotherme unter DSK-Analyse unter Verwendung von 5 °C/min von etwa (209 - 211 °C) zeigt.

11. Zolpidem-Salz nach irgendeinem der vorangehenden Ansprüche, das aus der Gruppe ausgewählt ist, die aus amorphem Zolpidemhydrogentartrat, amorphem Zolpidemtartrat, kristallinem Zolpidemhydrochlorid-Anhydrat, amorphem Zolpidemhydrochlorid, kristallinem Zolpidemhydrochlorid-Monohydrat, kristallinem Zolpidemhydrochlorid-Anhydrat, kristallinem Zolpidemhydrochlorid-Hemiethanolat, kristallinem Zolpidemhydrogensulfat und kristallinem Zolpidemmesylat besteht.

12. Pharmazeutische Zusammensetzung, umfassend eine pharmazeutisch wirksame Konzentration einer Zolpidem-Salzform, wie in irgendeinem der vorangehenden Ansprüche 1 - 11 beansprucht, und einen pharmazeutisch annehmbaren Träger oder ein pharmazeutisch annehmbares Verdünnungsmittel.

13. Zusammensetzung nach Anspruch 12, in der die Zusammensetzung eine feste Dosierungsform ist.

14. Zusammensetzung nach Anspruch 13, in der das Zolpidem-Salz in einer Menge im Bereich von etwa 5 mg bis 50 mg pro Dosiseinheit enthalten ist.

15. Pharmazeutische Zusammensetzung, umfassend einen pharmazeutisch annehmbaren flüssigen Träger, in dem eine wirksame Schlaf-induzierende Menge des Zolpidem-Salzes nach irgendeinem der Ansprüche 1 - 11 gelöst ist.

16. Flüssige pharmazeutische Zusammensetzung nach Anspruch 15, in der der flüssige Träger eine wässrige Lösung ist.

17. Verfahren, welches das Lösen einer Zolpidem-Salzform nach irgendeinem der Ansprüche 1 - 11 in einem Lösungsmittel umfasst.

18. Verfahren nach Anspruch 17, in dem das Lösungsmittel ein pharmazeutisch annehmbarer Träger oder ein pharmazeutisch annehmbares Verdünnungsmittel ist.

19. Verfahren, welches umfasst:
Vereinigen von Zolpidemhydrogentartrat nach Anspruch 8 mit einer freien Zolpidem-Base in einem Molverhältnis von 1 : 1 in Methanol, um eine methanolische Lösung mit einer Temperatur von mindestens 50 °C zu bilden; und Abkühlen der methanolischen Lösung, um festes Zolpidemtartrat zu fällen.

20. Verfahren zur Bereitstellung von amorphem Zolpidemtartrat, umfassend die Schritte: Mischen von Zolpidemhydrogentartrat nach Anspruch 8 mit freier Zolpidem-Base in einem Molverhältnis von etwa 1 : 1 in einem Lösungsmittel und Abtrennen des festen Produkts durch Trocknen.

21. Verfahren zur Bereitstellung eines Zolpidem-Salzes nach den Ansprüchen 1 - 11, umfassend die Schritte: Zusammenmischen von freier Zolpidem-Base und einer Säure in einem Lösungsmittel unter reaktiven Bedingungen, mit der Maßgabe, dass Methanol nicht als Lösungsmittel verwendet wird, wenn Zolpidemtartrat hergestellt wird.

22. Zolpidem-Salz, erhältlich nach Anspruch 21, wobei das Zolpidem-Salz eine ausreichende physikalische Stabilität aufweist, damit es beim Erwärmen von etwa 20 °C auf etwa 250 °C mit einer Geschwindigkeit von 5 °C/Minute keine Schmelzendotherme zeigt, welche freier Zolpidem-Base entspricht.

23. Verwendung eines Zolpidem-Salzes nach irgendeinem der Ansprüche 1 - 11, 22 zur Herstellung eines Medikaments.

24. Verwendung eines Zolpidem-Salzes nach irgendeinem der Ansprüche 1 - 11, 22 zur Herstellung eines Medikaments zur Behandlung von Schlafstörungen.

25. Verwendung eines ersten Zolpidem-Salzes nach irgendeinem der Ansprüche 1 bis 11, 22 bei der Herstellung eines separaten Zolpidem-Salzes.

## Revendications

1. Sel de zolpidem, qui est choisi dans le groupe du chlorhydrate de zolpidem, du chlorhydrate monohydrate de zolpidem, du chlorhydrate éthanolate de zolpidem, du méthanesulfonate (mésylate) de zolpidem, du tosylate de zolpidem, du maléate de zolpidem, de l'acétate de zolpidem, du trifluoroacétate de zolpidem, du bromhydrate de zolpidem, du fumarate de zolpidem, de l'hydrogénosulfate de zolpidem, et de l'hydrogéno-tartrate de zolpidem.

2. Sel de zolpidem selon la revendication 1, dans lequel le rapport molaire du fragment zolpidem à l'anion est dans la plage d'environ 0,9-1,35:1.

3. Sel de zolpidem selon les revendications 1 ou 2, dans lequel ledit rapport molaire du fragment zolpidem à l'anion est d'environ 1:1.

4. Sel de zolpidem selon l'une quelconque des revendications précédentes, dans lequel ladite forme de sel manifeste une seule endothermie de fusion quand elle est chauffée.

5. Sel de zolpidem selon l'une quelconque des revendications précédentes, dans lequel la phase solide est amorphe.

6. Sel de zolpidem selon l'une quelconque des revendications précédentes, dans lequel la phase solide est cristalline.

7. Sel de zolpidem selon l'une quelconque des revendications précédentes, dans lequel ledit sel est substantiellement dépourvu d'eau liée et de solvant organique.

8. Sel de zolpidem selon l'une quelconque des revendications précédentes, dans lequel ledit sel est l'hydrogénotartrate de zolpidem, le chlorhydrate de zolpidem, le mésylate de zolpidem, le tosylate de zolpidem, et l'hydrogénosulfate de zolpidem.

9. Sel de zolpidem selon la revendication 8 qui est l'hydrogénotartrate de zolpidem.

10. Sel de zolpidem selon la revendication 9, dans lequel ladite forme de sel est cristalline et a un point de fusion à environ (203-204°C) et/ou manifeste une seule exothermie de fusion, en analyse DSC en utilisant 5°C/min, d'environ (209-211 °C).

11. Sel de zolpidem selon l'une quelconque des revendications précédentes, choisi dans le groupe composé de l'hydrogénotartrate de zolpidem amorphe, le tartrate de zolpidem amorphe, le chlorhydrate anhydrate de zolpidem cristallin, le chlorhydrate de zolpidem amorphe, le chlorhydrate monohydrate de zolpidem cristallin, le chlorhydrate anhydrate de zolpidem cristallin, le chlorhydrate hémi-éthanoate de zolpidem cristallin, l'hydrogénosulfate de zolpidem cristallin, et le mésylate de zolpidem cristallin.

12. Composition pharmaceutique comprenant une concentration pharmaceutiquement efficace d'une forme de sel de zolpidem telle que revendiquée dans l'une quelconque des revendications 1 à 11 précédentes, et un véhicule ou un diluant pharmaceutiquement acceptable.

13. Composition selon la revendication 12, dans laquelle ladite composition est une forme pharmaceutique solide.

14. Composition selon la revendication 13, dans laquelle ledit sel de zolpidem est contenu en une quantité dans la plage d'environ 5 à 50 mg par dose unitaire.

15. Composition pharmaceutique comprenant un véhicule liquide pharmaceutiquement acceptable et contenant, à l'état dissous, une quantité somnifère efficace du sel de zolpidem selon l'une quelconque des revendications 1 à 11.

16. Composition pharmaceutique liquide selon la revendication 15, dans laquelle ledit véhicule liquide est une solution aqueuse.

17. Méthode, qui consiste à dissoudre une forme de sel de zolpidem selon l'une quelconque des revendications 1 à 11 dans un solvant.

18. Méthode selon la revendication 17, dans laquelle ledit solvant est un véhicule ou un diluant pharmaceutiquement acceptable.

19. Procédé qui consiste à :
combiner l'hydrogénotartrate de zolpidem de la revendication 8 avec une base libre de zolpidem dans un rapport molaire de 1:1 dans du méthanol pour former une solution méthanolique ayant une température d'au moins 50°C ; et à refroidir ladite solution méthanolique pour précipiter le tartrate de zolpidem solide.

20. Procédé pour préparer un tartrate de zolpidem amorphe comprenant les étapes consistant à mélanger dans un solvant l'hydrogénotartrate de zolpidem de la revendication 8 avec une base libre de zolpidem dans un rapport molaire d'environ 1:1 et à séparer le produit solide par séchage.

21. Procédé pour préparer un sel de zolpidem selon les revendications 1 à 11, comprenant les étapes consistant à mélanger ensemble dans un solvant, dans des conditions réactives, une base libre de zolpidem et un acide, à condition que le méthanol ne soit pas utilisé comme solvant quand on prépare un tartrate de zolpidem.

22. Sel de zolpidem pouvant être obtenu selon la revendication 21, ce sel de zolpidem ayant une stabilité physique suffisante pour que, après chauffage entre environ 20 et environ 250°C à une vitesse de 5°C/minute, il ne manifeste pas d'endothermie de fusion qui corresponde à la base libre du zolpidem.

23. Utilisation d'un sel de zolpidem selon l'une quelconque des revendications 1 à 11, 22 pour préparer un médicament.

24. Utilisation d'un sel de zolpidem selon l'une quelconque des revendications 1 à 11, 22 dans la fabrication d'un médicament pour le traitement des troubles du sommeil.

25. Utilisation d'un premier sel de zolpidem selon l'une quelconque des revendications 1 à 11, 22 dans la fabrication d'un sel de zolpidem séparé.
